# EUROPEAN PATENT APPLICATION

(11) **EP 4 497 408 A1**
(43) Date of publication of application: **29.01.2025**
(21) Application number: 23819871.7
(22) Date of filing: 07.06.2023
(51) Int. Cl.: A61B 34/37

(54) **SURGICAL SYSTEM**

(30) Priority: 09.06.2022 JP 2022093612
(71) Applicant: KAWASAKI JUKOGYO KABUSHIKI KAISHA, Kobe-shi, Hyogo 650-8670 (JP)
(72) Inventor: TAKAHASHI, Tsuyoshi, Kobe-shi, Hyogo 650-0047 (JP); SASAMORI, Kazuya, Kobe-shi, Hyogo 650-0047 (JP); KODAMA, Kazuki, Kobe-shi, Hyogo 650-8670 (JP)
(74) Representative: Hoffmann Eitle
(86) International application number: PCT/JP2023/021166
(87) International publication number: WO 2023/238891

(57) **Abstract**

A controller (8b, 31) of a surgical system (100) is configured or programmed to acquire a distal end position of a surgical instrument (4), acquire a second imaging range (A2) reflecting a positional deviation related to the distal end position of the surgical instrument (4) and a diameter of a shaft of the surgical instrument with respect to a first imaging range (A1) corresponding to an angle of view of an endoscope (6), and determine whether or not the distal end position of the surgical instrument (4) is within the second imaging range (A2).

## Description

### Technical Field

The present invention relates to a surgical system, and more particularly, it relates to a surgical system including a plurality of manipulators each supporting an endoscope or a surgical instrument.

### Background Art

Conventionally, a surgical system including a plurality of manipulators each supporting an endoscope or a surgical instrument is known. Such a surgical system is disclosed in Japanese Patent Laid-Open No. 2013-188574, for example.

Japanese Patent Laid-Open No. 2013-188574 discloses a surgery system including a plurality of manipulators each supporting an endoscope or a tool (surgical instrument), and a controller. In the surgical system disclosed in Japanese Patent Laid-Open No. 2013-188574, the controller determines whether or not the current position of a distal end of the tool (surgical instrument) is outside the field of view of the endoscope, and displays information about the tool that is outside the field of view as a symbol (sign) in a boundary area outside a display area of a monitor screen.

### Prior Art

### Patent Document

Patent Document 1: Japanese Patent Laid-Open No. 2013-188574

### Summary of the Invention

### Problems to be Solved by the Invention

However, in the surgical system disclosed in Japanese Patent Laid-Open No. 2013-188574, the distal end position of the tool recognized by the controller may deviate significantly from the actual position due to a manufacturing error in a distance between the manipulator supporting the endoscope and the manipulator supporting the tool (surgical instrument), a positional deviation of a field of view range of the endoscope, a positional deviation of the distal end position of the tool, etc. In such a case, the tool may not be able to be found even when the endoscope is pointed in a direction indicated by the symbol (sign). Furthermore, an operator may have difficulty finding the tool depending on the positional relationship between the endoscope and the tool. In addition, it is necessary to inform the operator whether or not the distal end position of the tool is outside the field of view such that the operator does not feel uncomfortable. Therefore, it may be difficult for the operator to easily confirm the position of the tool (surgical instrument).

The present invention is intended to solve the above problems. The present invention aims to provide a surgical system that allows an operator to easily confirm the position of a surgical instrument even when the surgical instrument is positionally deviated.

### Means for Solving the Problems

A surgical system according to a first aspect of the present invention includes a first manipulator to support an endoscope, a second manipulator to support a surgical instrument, and a controller including one or more processors. The controller is configured or programmed to acquire a distal end position of the surgical instrument, acquire a second imaging range reflecting a positional deviation related to the distal end position of the surgical instrument and a diameter of a shaft of the surgical instrument with respect to a first imaging range corresponding to an angle of view of the endoscope, and determine whether or not the distal end position of the surgical instrument is within the second imaging range.

In the surgical system according to the first aspect of the present invention, as described above, the controller is configured or programmed to acquire the distal end position of the surgical instrument, acquire the second imaging range reflecting the positional deviation related to the distal end position of the surgical instrument and the diameter of the shaft of the surgical instrument with respect to the first imaging range corresponding to the angle of view of the endoscope, and determine whether or not the distal end position of the surgical instrument is within the second imaging range. Accordingly, whether or not the distal end position of the surgical instrument is outside the field of view of the endoscope can be determined based on the second imaging range reflecting the positional deviation related to the distal end position of the surgical instrument. Consequently, when the distal end position of the surgical instrument with respect to the field of view of the endoscope is uncertain due to the distal end position of the surgical instrument being deviated with respect to the field of view of the endoscope, the possibility is reduced or prevented that the direction of the uncertain distal end position of the surgical instrument is indicated. Furthermore, whether or not the distal end position of the surgical instrument is outside the field of view of the endoscope can be determined based on the second imaging range reflecting the diameter of the shaft of the surgical instrument, and thus it is possible to determine whether or not the distal end position of the surgical instrument having some size, not just a specific point at the distal end of the surgical instrument, is within the field of view of the endoscope. Consequently, even when the surgical instrument is positionally deviated, the operator can easily confirm the position of the surgical instrument.

A surgical system according to a second aspect of the present invention includes a first manipulator to support an endoscope, a second manipulator to support a surgical instrument, and a controller including one or more processors. The controller is configured or programmed to control the first manipulator to move the endoscope about a first pivot position as a fulcrum, and control the second manipulator to move the surgical instrument about a second pivot position as a fulcrum, and the controller is configured or programmed to acquire a distal end position of each of the endoscope and the surgical instrument, and display an indication prompting an operator to pull the endoscope toward a proximal end side of the endoscope when a distance between the distal end position of the surgical instrument and the first pivot position is smaller than a distance between the distal end position of the endoscope and the first pivot position.

In the surgical system according to the second aspect of the present invention, as described above, the controller is configured or programmed to acquire the distal end position of each of the endoscope and the surgical instrument, and display an indication prompting the operator to pull the endoscope toward the proximal end side of the endoscope when the distance between the distal end position of the surgical instrument and the first pivot position is smaller than the distance between the distal end position of the endoscope and the first pivot position. Accordingly, when the distance between the distal end position of the surgical instrument and the first pivot position is smaller than the distance between the distal end position of the endoscope and the first pivot position, and the distal end position of the surgical instrument does not enter the field of view of the endoscope even when the orientation of the endoscope is changed, an indication is displayed prompting the operator to pull the endoscope toward the proximal end side of the endoscope. Thus, the operator can easily find the distal end position of the surgical instrument outside the field of view by pulling the endoscope toward the proximal end side of the endoscope. Consequently, even when the surgical instrument is positionally deviated, the operator can easily confirm the position of the surgical instrument.

### Effect of the Invention

According to the present invention, even when the surgical instrument is positionally deviated, the operator can easily confirm the position of the surgical instrument.

### Brief Description of the Drawings

FIG. 1 is a diagram showing the configuration of a surgical system according to an embodiment.
FIG. 2 is a diagram showing the configuration of a medical manipulator according to the embodiment.
FIG. 3 is a diagram showing the configuration of an operation handle according to the embodiment.
FIG. 4 is a diagram showing the configuration of foot pedals according to the embodiment.
FIG. 5 is a diagram showing the configuration of an arm of the medical manipulator according to the embodiment.
FIG. 6 is a diagram showing a pair of forceps.
FIG. 7 is a perspective view showing the configuration of an operation unit of the medical manipulator according to the embodiment.
FIG. 8 is a diagram showing an endoscope.
FIG. 9 is a diagram showing a pivot position teaching instrument.
FIG. 10 is a diagram for illustrating translational movement of the arm.
FIG. 11 is a diagram for illustrating rotational movement of the arm.
FIG. 12 is a block diagram showing the configuration of a control unit of the medical manipulator according to the embodiment.
FIG. 13 is a diagram showing an image captured by the endoscope and a graphical user interface.
FIG. 14 is a diagram showing graphical user interface areas.
FIG. 15 is a diagram for illustrating a display of a clutch area.
FIG. 16 is a diagram for illustrating a display of a medical instrument usage information area.
FIG. 17 is a diagram for illustrating a display of a left pop-up area.
FIG. 18 is a diagram for illustrating a display of a right pop-up area.
FIG. 19 is a diagram for illustrating a display on a touch panel of a remote control apparatus.
FIG. 20 is a diagram for illustrating marks according to the embodiment.
FIG. 21 is a diagram for illustrating reflection of interference correction between arms.
FIG. 22 is a diagram for illustrating positional deviations of the pair of forceps and the endoscope.
FIG. 23 is a table showing an example of the insertion amount and deviation angles of the pair of forceps and the endoscope.
FIG. 24 is a first diagram showing an example of a deviation direction relationship between the pair of forceps and the endoscope.
FIG. 25 is a second diagram showing an example of a deviation direction relationship between the pair of forceps and the endoscope.
FIG. 26 is a diagram showing an example of a relationship between the imaging range of the endoscope and a deviation.
FIG. 27 is a diagram showing a first imaging range and a second imaging range of the endoscope.
FIG. 28 is a diagram showing an example in which the distal end position of the pair of forceps and the pivot position of the endoscope are close to each other.
FIG. 29 is a diagram showing an example in which the distal end position of the pair of forceps and the pivot position of the endoscope are far apart from each other.
FIG. 30 is a flowchart for illustrating an out-of-field-of-view determination process according to the embodiment.

### Modes for Carrying Out the Invention

An embodiment embodying the present invention is hereinafter described on the basis of the drawings.

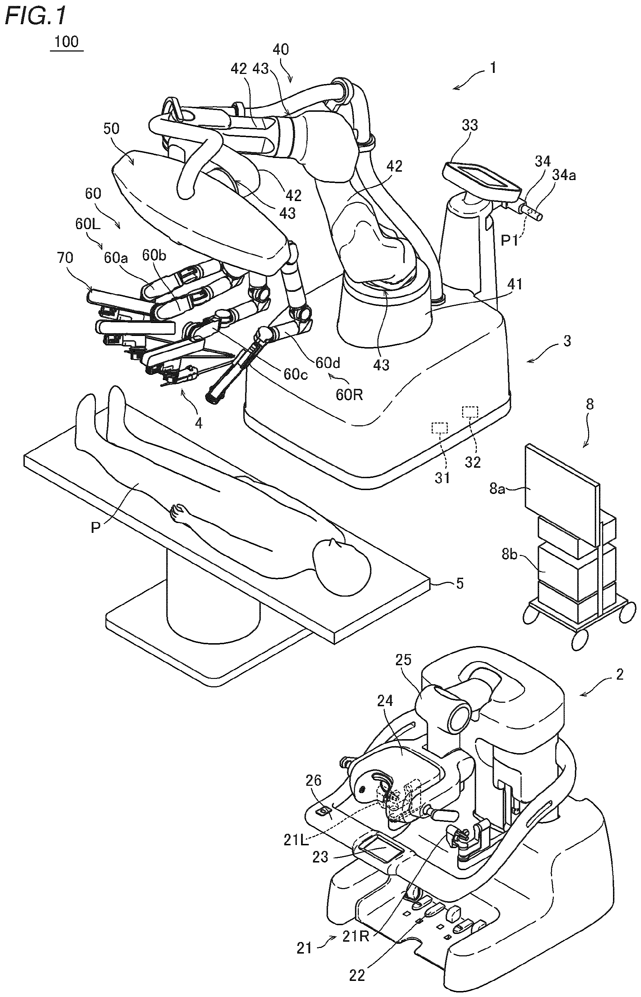

The configuration of a surgical operation system 100 according to the embodiment is now described with reference to FIGS. 1 to 30. The surgical operation system 100 includes a medical manipulator 1 that is a patient P-side apparatus, and a remote control apparatus 2 that is an operator-side apparatus to operate the medical manipulator 1. The medical manipulator 1 includes a medical cart 3 and is movable. The remote control apparatus 2 is spaced apart from the medical manipulator 1, and the medical manipulator 1 is remotely operated by the remote control apparatus 2. An operator (such as a doctor) inputs a command to the remote control apparatus 2 to cause the medical manipulator 1 to perform a desired operation. The remote control apparatus 2 transmits the input command to the medical manipulator 1. The medical manipulator 1 operates based on the received command. The medical manipulator 1 is arranged in an operating room that is a sterilized sterile field. The surgical operation system 100 is an example of a surgical system in the claims.

The remote control apparatus 2 is arranged inside or outside the operating room, for example. The remote control apparatus 2 includes operation handles 21, foot pedals 22, a touch panel 23, a monitor 24, a support arm 25, and a support bar 26. The operation handles 21 include operation handles for the operator (such as a doctor) to input commands.

The operation handles 21 are configured to operate medical instruments 4. The operation handles 21 receive the amounts of operation for the medical instruments 4. The operation handles 21 include an operation handle 21L that is located on the left side as viewed from the operator (such as a doctor) and is to be operated by the left hand of the operator, and an operation handle 21R that is located on the right side and is to be operated by the right hand of the operator.

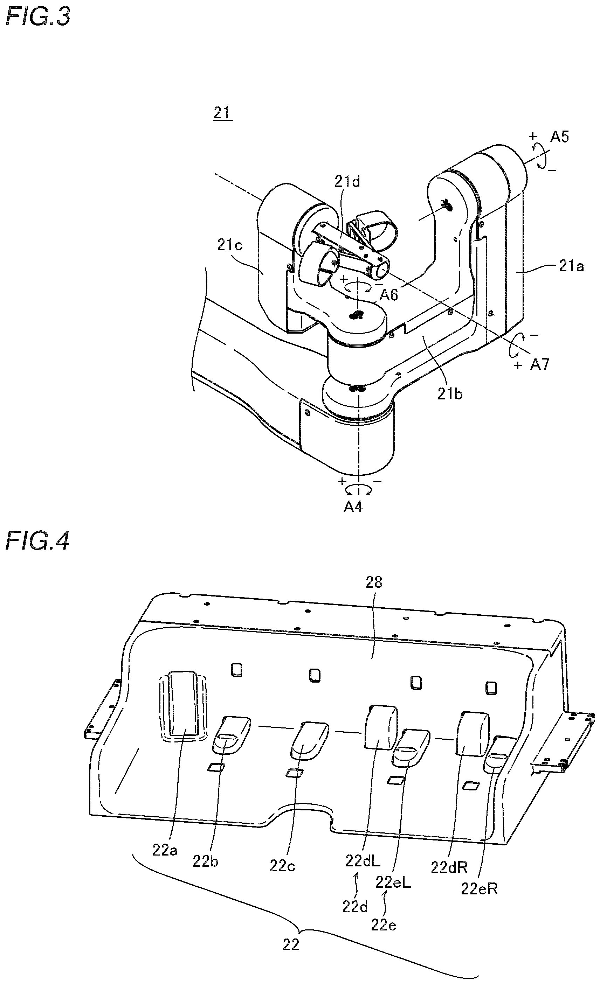

As shown in FIG. 3, each of the operation handles 21 includes a link 21a, a link 21b, a link 21c, and a link 21d that is to be operated by the operator (such as a doctor). The link 21a is rotatable about an A4 axis. By rotating the link 21a about the A4 axis, an arm portion 61 described below is rotated about a JT4 axis. The link 21b is rotatable about an A5 axis with respect to the link 21a. By rotating the link 21b about the A5 axis, the arm portion 61 described below is rotated about a JT5 axis. The link 21c is rotatable about an A6 axis with respect to the link 21b. By rotating the link 21c about the A6 axis, the arm portion 61 is rotated about a JT6 axis. The link 21d is rotatable about an A7 axis with respect to the link 21c. By rotating the link 21d about the A7 axis, the arm portion 61 is rotated about a JT7 axis. The medical instruments 4 are examples of a "surgical instrument" in the claims.

In the operation handles 21, the movement amounts of arms 60 (medial instruments 4) are changed (scaled) with respect to operation amounts received by the operation handles 21. For example, when the scale factor of the movement amounts is set to 1/2, the medical instruments 4 are controlled to move 1/2 of the movement distance of the operation handles 21. Thus, fine surgery can be performed accurately.

As shown in FIG. 4, a plurality of foot pedals 22 are provided to perform functions related to the medical instruments 4. The plurality of foot pedals 22 are arranged on a base 28. The foot pedals 22 include a switching pedal 22a, a clutch pedal 22b, a camera pedal 22c, incision pedals 22d, and coagulation pedals 22e. The switching pedal 22a, the clutch pedal 22b, the camera pedal 22c, the incision pedals 22d, and the coagulation pedals 22e are operated by the foot of the operator. The incision pedals 22d include an incision pedal 22dR for a right arm 60, and an incision pedal 22dL for a left arm 60. The coagulation pedals 22e includes a coagulation pedal 22eR for the right arm 60 and a coagulation pedal 22eL for the left arm 60. The camera pedal 22c is an example of an "input" or a "third switch" in the claims.

The switching pedal 22a switches arms 60 to be operated by the operation handles 21. In this embodiment, the clutch pedal 22b performs a clutch operation to temporarily disconnect an operation connection between the arms 60 and the operation handles 21. While the clutch pedal 22b is being pressed by the operator, operations by the operation handles 21 are not transmitted to the arms 60.

The camera pedal 22c is operated to allow the two operation handles 21 to move an endoscope 6. Specifically, the camera pedal 22c is provided to input a command for moving the endoscope 6. More specifically, when the camera pedal 22c is pressed by the operator, the command for moving the endoscope 6 is input. When the command that enables the endoscope 6 to move is input by the camera pedal 22c (that is, while the camera pedal 22c is being pressed by the operator), the endoscope 6 is able to be moved by operating both the operation handle 21R and the operation handle 21L.

While the incision pedals 22d (coagulation pedals 22e) are being pressed by the operator, a high-frequency current for incising or coagulating tissue flows from an electrosurgical device (not shown) to the medical instruments 4 (electrocauteries).

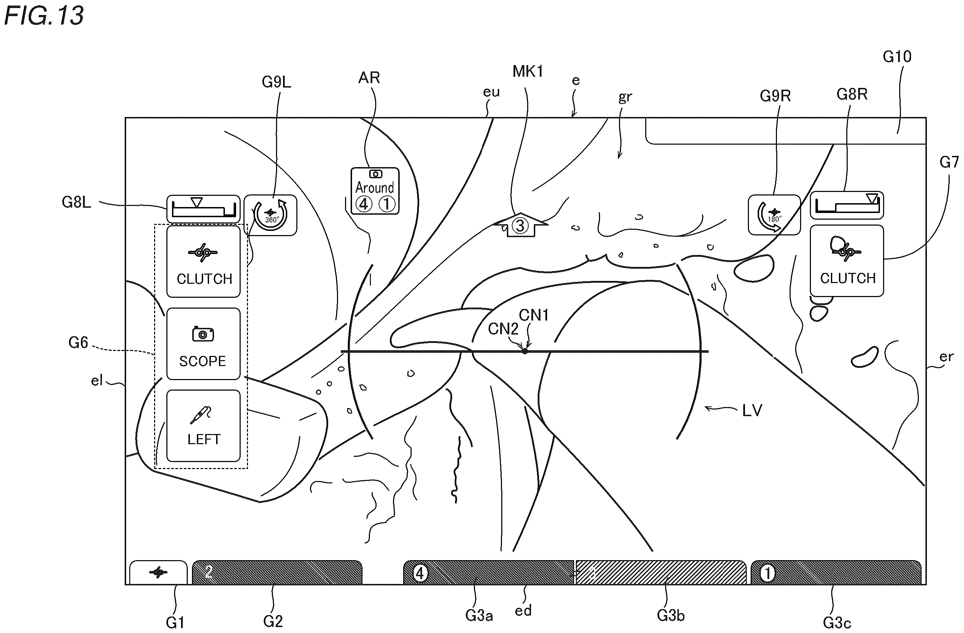

As shown in FIG. 1, the monitor 24 is a scope-type display that displays an endoscopic image (see FIG. 13) captured by the endoscope 6. The support arm 25 supports the monitor 24 so as to align the height of the monitor 24 with the height of the face of the operator (such as a doctor). The touch panel 23 is arranged on the support bar 26. The head of the operator is detected by a sensor (not shown) provided in the vicinity of the monitor 24 such that the medical manipulator 1 can be operated by the remote control apparatus 2. The operator operates the operation handles 21 and the foot pedals 22 while visually recognizing an affected area on the monitor 24. Thus, a command is input to the remote control apparatus 2. The command input to the remote control apparatus 2 is transmitted to the medical manipulator 1.

The medical cart 3 includes a control unit 31 that controls the operation of the medical manipulator 1 and a storage 32 that stores programs for controlling the operation of the medical manipulator 1, etc. Based on the command input to the remote control apparatus 2, the control unit 31 of the medical cart 3 controls the operation of the medical manipulator 1.

The medical cart 3 also includes an input 33. The input 33 receives operations to move a positioner 40, an arm base 50, and a plurality of arms 60 or change their postures mainly in order to prepare for surgery before the surgery.

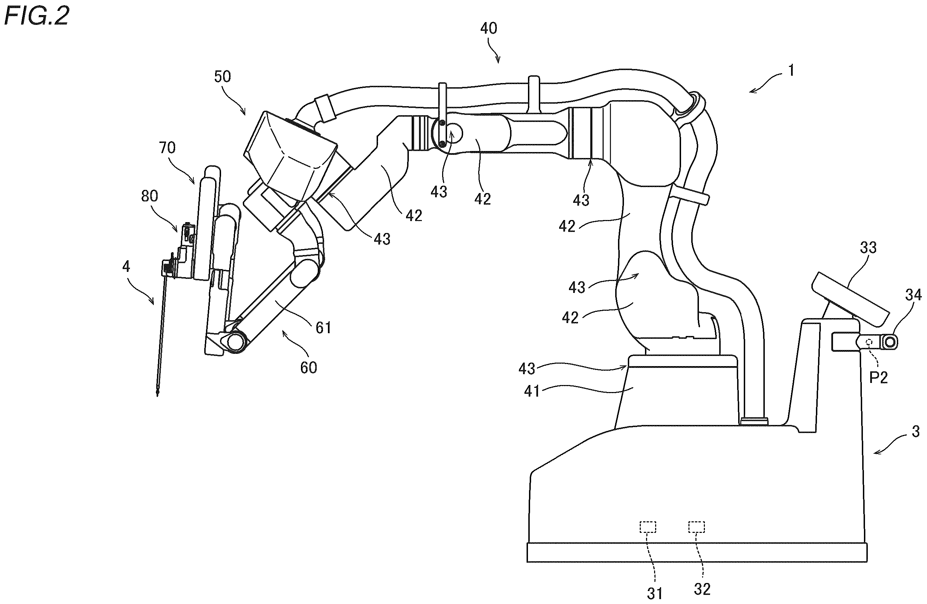

The medical manipulator 1 shown in FIGS. 1 and 2 is arranged in the operating room. The medical manipulator 1 includes the medical cart 3, the positioner 40, the arm base 50, and the plurality of arms 60. The arm base 50 is attached to a distal end of the positioner 40. The arm base 50 has a relatively long rod shape (elongated shape). The bases of the plurality of arms 60 are attached to the arm base 50. Each of the plurality of arms 60 is able to take a folded (stored) posture. The arm base 50 and the plurality of arms 60 are covered with sterile drapes (not shown) and used. The arms 60 support the medical instruments 4.

The positioner 40 includes a 7-axis articulated robot, for example. The positioner 40 is arranged on the medical cart 3. The positioner 40 moves the arm base 50. Specifically, the positioner 40 moves the position of the arm base 50 three-dimensionally.

The positioner 40 includes a base 41 and a plurality of links 42 coupled to the base 41. The plurality of links 42 are coupled to each other by joints 43.

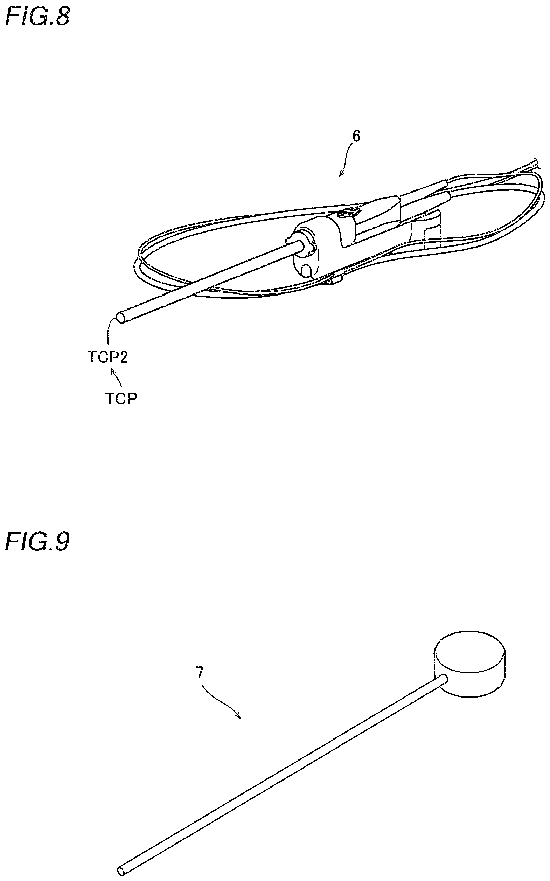

As shown in FIG. 1, the medical instrument 4 is attached to a distal end of each of the plurality of arms 60. The medical instrument 4 includes a replaceable instrument or the endoscope 6 (see FIG. 8) to capture an image of a surgical site, for example.

The surgical operation system 100 includes a monitor cart 8, as shown in FIG. 1. The monitor cart 8 includes a display 8a. The display 8a is provided separately from the monitor 24 of the remote control apparatus 2. The monitor cart 8 includes an image processor 8b. The same image as that displayed on the monitor 24 of the remote control apparatus 2 is displayed on the display 8a of the monitor cart 8. That is, the image displayed on the monitor 24 and viewed by the operator can be viewed by an operator (such as a nurse or an assistant) around the medical manipulator 1 and a patient P on the display 8a of the monitor cart 8. The image processor 8b is an example of a "controller" in the claims.

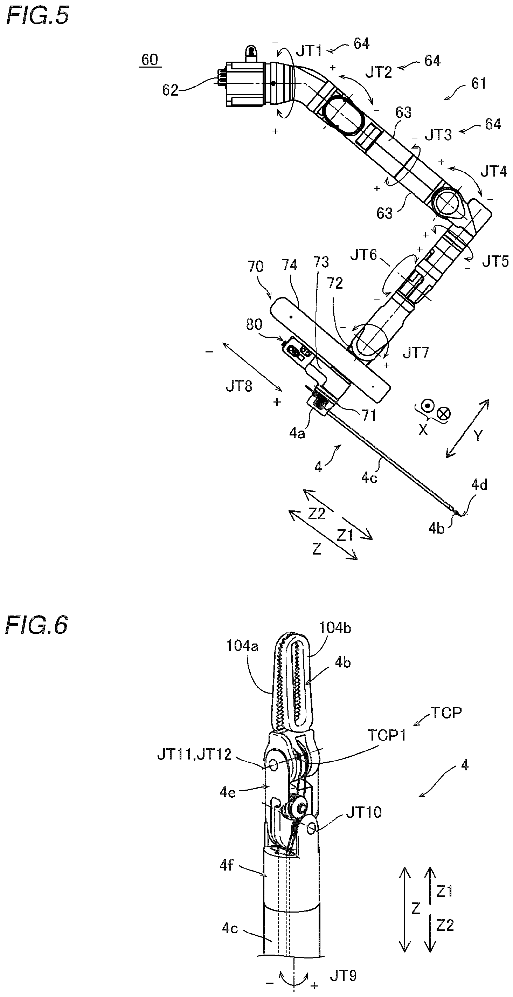

As shown in FIG. 5, the instrument includes a driven unit 4a driven by servomotors M2 provided in a holder 71 of each of the arms 60. A pair of forceps 4b is provided at a distal end of the instrument.

As shown in FIG. 6, the instrument includes a first support 4e that supports the proximal end sides of end effector members 104a and 104b such that the proximal end sides of the end effector members 104a and 104b are rotatable about a JT11 axis on the distal end sides, a second support 4f that supports the proximal end side of the first support 4e such that the proximal end side of the first support 4e is rotatable about a JT10 axis on the distal end side, and a shaft 4c connected to the proximal end side of the second support 4f. The driven unit 4a, the shaft 4c, the second support 4f, the first support 4e, and the pair of forceps 4b are arranged along a Z direction. The JT11 axis is orthogonal to a direction (Z direction) in which the shaft 4c extends. The JT10 axis is spaced apart from the JT11 axis in the direction in which the shaft 4c extends, and is orthogonal to the direction in which the shaft 4c extends and the JT11 axis.

The pair of forceps 4b is attached to the first support 4e so as to rotate about the JT11 axis. The second support 4f supports the first support 4e such that the first support 4e is rotatable about the JT10 axis. That is, the first support 4e is attached to the second support 4f so as to rotate about the JT10 axis. A portion of the first support 4e on the distal end side (Z1 direction side) has a U-shape. A tool center point (TCP1, clevis) is set at the center of a distal end of the U-shaped portion of the first support 4e in the JT11 axis.

The medical instrument 4 (pair of forceps 4b) includes a JT9 axis as a rotation axis (an axis along the direction in which the shaft 4c extends) of the shaft 4c, and a JT12 axis as an opening/closing axis of the pair of forceps 4b. A plurality of (four, for example) servomotors M2 provided in the holder 71 of the arm 60 are provided, and rotary bodies of the driven unit 4a are driven by the plurality of servomotors M2. Thus, the medical instrument 4 is driven around the J9 to J12 axes.

As shown in FIG. 8, a TCP2 of the endoscope 6 is set at a distal end of the endoscope 6.

The configuration of the arm 60 is now described in detail.

As shown in FIG. 5, the arm 60 includes the arm portion 61 (a base 62, links 63, and joints 64) and a translation mechanism 70 provided at a distal end of the arm portion 61. The distal end side of the arm 60 three-dimensionally moves with respect to the base side (arm base 50) of the arm 60. The arm portion 61 includes a 7-axis articulated robot arm. The plurality of arms 60 have the same or similar configuration as each other.

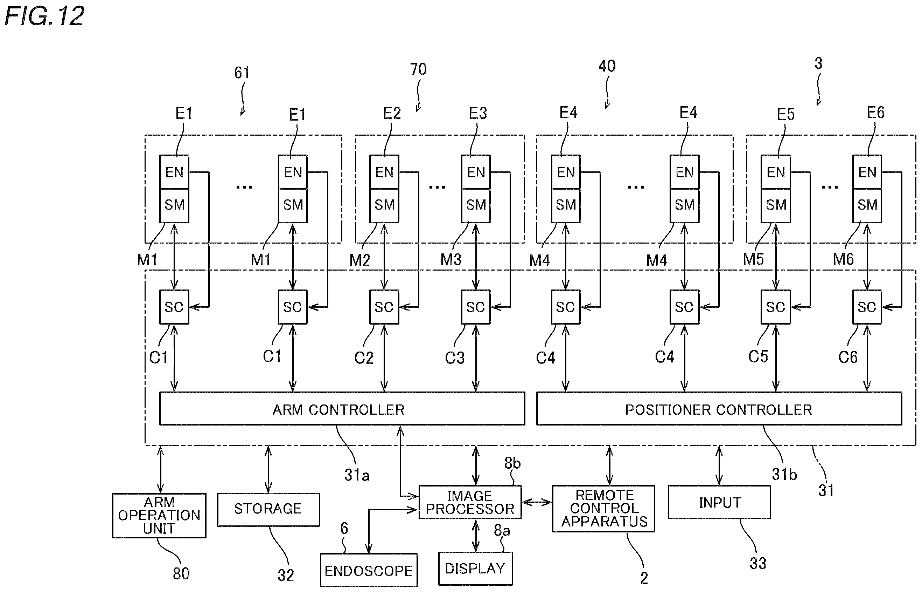

As shown in FIG. 5, the arm 60 includes JT1 to JT7 axes as rotation axes and a J8 axis as a linear motion axis. The JT1 to JT7 axes correspond to the rotation axes of the joints 64 of the arm portion 61. The JT7 axis also corresponds to a proximal end side link 72 of the translation mechanism 70. The JT8 axis corresponds to an axis for moving a distal end side link 73 of the translation mechanism 70 relative to the proximal end side link 72 along the Z direction. That is, servomotors M1 shown in FIG. 12 are provided so as to correspond to the JT1 to JT7 axes of the arm 60. A servomotor M3 is provided so as to correspond to the JT8 axis.

The translation mechanism 70 is provided on the distal end side of the arm portion 61, and the medical instrument 4 is attached thereto. The translation mechanism 70 translates the medical instrument 4 in a direction in which the medical instrument 4 is inserted into the patient P. Furthermore, the translation mechanism 70 translates the medical instrument 4 relative to the arm portion 61. Specifically, the translation mechanism 70 includes the holder 71 that holds the medical instrument 4. The servomotors M2 (see FIG. 12) are housed in the holder 71.

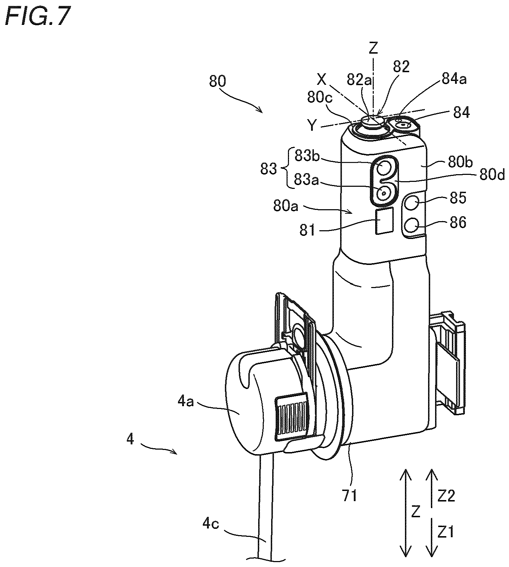

As shown in FIG. 7, the medical manipulator 1 includes an arm operation unit 80 attached to each of the arms 60 to operate the arm 60. The arm operation unit 80 includes an enable switch 81, a joystick 82, and a switch unit 83. The enable switch 81 enables or disables movement of the arm 60 in response to the joystick 82 and the switch unit 83. The enable switch 81 enables movement of the medical instrument 4 by the arm 60 when the enable switch 81 is pressed by the operator (such as a nurse or an assistant) grasping the arm operation unit 80. The joystick 82 and the switch unit 83 are used to operate the arm 60. The enable switch 81 is an example of an "input" or a "second switch" in the claims.

The switch unit 83 includes a switch 83a to move the medical instrument 4 in the direction in which the medical instrument 4 is inserted into the patient P along the longitudinal direction of the medical instrument 4, and a switch 83b to move the medical instrument 4 in a direction opposite to the direction in which the medical instrument 4 is inserted into the patient P. Both the switch 83a and the switch 83b are push-button switches.

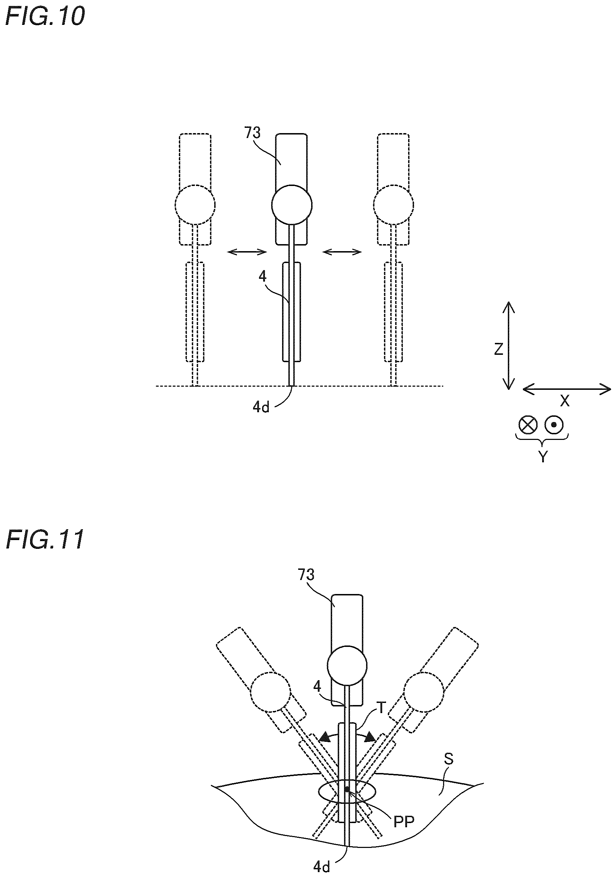

As shown in FIG. 7, the arm operation unit 80 includes a pivot button 85 to teach a pivot position PP that serves as a fulcrum (see FIG. 11) for movement of the medical instrument 4 attached to the arm 60. The pivot button 85 is provided adjacent to the enable switch 81 on a surface 80b of the arm operation unit 80. When the pivot button 85 is pressed while the distal end of the endoscope 6 (see FIG. 8) or a distal end of a pivot position teaching instrument 7 (FIG. 9) is moved to a position corresponding to the insertion position of a trocar T inserted into the body surface S of the patient P, the pivot position PP is taught and stored in the storage 32. In the teaching of the pivot position PP, the pivot position PP is set as one point (coordinates), and the direction of the medical instrument 4 is not set.

As shown in FIG. 1, the endoscope 6 is attached to one (arm 60c, for example) of the plurality of arms 60, and medical instruments 4 other than the endoscope 6 are attached to the remaining arms 60 (arms 60a, 60b, and 60d, for example). Specifically, in surgery, the endoscope 6 is attached to one of four arms 60, and the medical instruments 4 (such as pairs of forceps) other than the endoscope 6 are attached to the three arms 60. The pivot position PP is taught with the endoscope 6 attached to the arm 60 to which the endoscope 6 is to be attached. Furthermore, the pivot position PP is taught with the pivot position teaching instrument 7 attached to the arm 60 to which the medical instrument 4 other than the endoscope 6 is to be attached. The endoscope 6 is attached to one of two arms 60 (arms 60b and 60c) arranged in the center among the four arms 60 arranged adjacent to each other. That is, the pivot position PP is individually set for each of the plurality of arms 60. The arm 60c is an example of a "first manipulator" in the claims. The arms 60a, 60b, and 60d are examples of a "second manipulator" in the claims.

As shown in FIG. 7, an adjustment button 86 is provided on the surface 80b of the arm operation unit 80 to optimize the position of the arm 60. After the pivot position PP for the arm 60 to which the endoscope 6 has been attached is taught, the adjustment button 86 is pressed such that the positions of the other arms 60 (arm base 50) are optimized.

As shown in FIG. 7, the arm operation unit 80 includes a mode switching button 84 to switch between a mode for translationally moving the medical instrument 4 attached to the arm 60 (see FIG. 10) and a mode for rotationally moving the medical instrument 4 (see FIG. 11). Furthermore, a mode indicator 84a is provided in the vicinity of the mode switching button 84. The mode indicator 84a indicates a switched mode. Specifically, the mode indicator 84a is on (rotational movement mode) or off (translational movement mode) to indicate a current mode (the translational movement mode or the rotational movement mode).

The mode indicator 84a also serves as a pivot position indicator that indicates that the pivot position PP has been taught.

As shown in FIG. 10, in the mode for translationally moving the arm 60, the arm 60 is moved such that a distal end 4d of the medical instrument 4 is moved in an X-Y plane. As shown in FIG. 11, in the mode for rotationally moving the arm 60, the arm 60 is moved such that the medical instrument 4 is rotationally moved about the pair of forceps 4b when the pivot position PP is not taught, and the medical instrument 4 is rotationally moved about the pivot position PP as a fulcrum when the pivot position PP is taught. In this case, the medical instrument 4 is rotationally moved with the shaft 4c of the medical instrument 4 inserted into the trocar T.

As shown in FIG. 12, the arm 60 includes a plurality of servomotors M1, encoders E1, and speed reducers (not shown) so as to correspond to a plurality of joints 64 of the arm portion 61. The encoders E1 detect rotation angles of the servomotors M1. The speed reducers slow down rotation of the servomotors M1 to increase the torques.

As shown in FIG. 12, the translation mechanism 70 includes the servomotors M2 to rotate the rotary bodies provided in the driven unit 4a of the medical instrument 4, the servomotor M3 to translationally move the medical instrument 4, encoders E2 and E3, and speed reducers (not shown). The encoders E2 and E3 detect rotation angles of the servomotors M2 and M3, respectively. The speed reducers slow down rotation of the servomotors M2 and M3 to increase the torques.

A plurality of servomotors M4, a plurality of encoders E4, and a plurality of speed reducers (not shown) are provided in the positioner 40 so as to correspond to a plurality of joints 43 of the positioner 40. The encoders E4 detect rotation angles of the servomotors M4. The speed reducers slow down rotation of the servomotors M4 to increase the torques.

The medical cart 3 includes servomotors M5 to drive a plurality of front wheels (not shown) of the medical cart 3, encoders E5, speed reducers (not shown), and brakes. The speed reducers slow down rotation of the servomotors M5 to increase the torques. A potentiometer P1 (see FIG. 1) is provided on a throttle 34a of the medical cart 3, and the servomotors M5 of the front wheels are driven based on a rotation angle detected by the potentiometer P1 according to the twist of the throttle 34a. Rear wheels (not shown) of the medical cart 3 are of the dual wheel type, and the rear wheels are steered based on rightward-leftward rotation of an operation handle 34. Furthermore, a potentiometer P2 (see FIG. 2) is provided on the operation handle 34 of the medical cart 3, and servomotors M6, encoders E6, and speed reducers (not shown) are provided on the rear wheels of the medical cart 3. The speed reducers slow down rotation of the servomotors M6 to increase the torques. The servomotors M6 are driven based on a rotation angle detected by the potentiometer P2 according to rightward-leftward rotation of the operation handle 34. That is, steering of the rear wheels by the rightward-leftward rotation of the operation handle 34 is power-assisted by the servomotors M6.

The front wheels of the medical cart 3 are driven such that the medical cart 3 moves in a forward-rearward direction. Furthermore, the operation handle 34 of the medical cart 3 is rotated such that the rear wheels are steered, and the medical cart 3 turns in a right-left direction.

The control unit 31 of the medical cart 3 includes an arm controller 31a to control movement of the plurality of arms 60 based on commands, and a positioner controller 31b to control movement of the positioner 40 and driving of the front wheels and rear wheels (not shown) of the medical cart 3 based on commands. Servo controllers C1 that control the servomotors M1 to drive the arm 60 are electrically connected to the arm controller 31a. The encoders E1 that detect the rotation angles of the servomotors M1 are electrically connected to the servo controllers C1. The control unit 31 is an example of a "controller" in the claims.

Servo controllers C2 that control the servomotors M2 to drive the medical instrument 4 are electrically connected to the arm controller 31a. The encoders E2 that detect the rotation angles of the servomotors M2 are electrically connected to the servo controllers C2. A servo controller C3 that controls the servomotor M3 to translate the translation mechanism 70 is electrically connected to the arm controller 31a. The encoder E3 that detects the rotation angle of the servomotor M3 is electrically connected to the servo controller C3.

An operation command input to the remote control apparatus 2 is input to the arm controller 31a. The arm controller 31a generates position commands based on the input operation command and the rotation angles detected by the encoders E1 (E2, E3), and outputs the position commands to the servo controllers C1 (C2, C3). The servo controllers C1 (C2, C3) generate torque commands based on the position commands input from the arm controller 31a and the rotation angles detected by the encoders E1 (E2, E3), and output the torque commands to the servomotors M1 (M2, M3). Thus, the arm 60 is moved according to the operation command input to the remote control apparatus **2.** The control unit 31 controls the arm 60c such that the endoscope 6 is moved about the pivot position PP as a fulcrum. The control unit 31 also controls the arm 60a (60b, 60d) such that the medical instrument 4 is moved about the pivot position PP as a fulcrum.

As shown in FIG. 12, the control unit 31 (arm controller 31a) operates the arm 60 based on an input signal from the joystick 82 of the arm operation unit 80. Specifically, the arm controller 31a generates position commands based on the input signal (operation command) input from the joystick 82 and the rotation angles detected by the encoders E1, and outputs the position commands to the servo controllers C1. The servo controllers C1 generate torque commands based on the position commands input from the arm controller 31a and the rotation angles detected by the encoders E1, and output the torque commands to the servomotors M1. Thus, the arm 60 is moved according to the operation command input to the joystick 82.

The control unit 31 (arm controller 31a) operates the arm 60 based on an input signal from the switch unit 83 of the arm operation unit 80. Specifically, the arm controller 31a generates a position command based on the input signal (operation command) input from the switch unit 83 and the rotation angle detected by the encoders E1 or the encoder E3, and outputs the position command to the servo controllers C1 or the servo controller C3. The servo controllers C1 or the servo controller C3 generates a torque command based on the position command input from the arm controller 31a and the rotation angle detected by the encoders E1 or the encoder E3, and outputs the torque command to the servomotors M1 or the servomotor M3. Thus, the arm 60 is moved according to the operation command input to the switch unit 83.

As shown in FIG. 12, servo controllers C4 that control the servomotors M4 to move the positioner 40 are electrically connected to the positioner controller 31b. The encoders E4 that detect the rotation angles of the servomotors M4 are electrically connected to the servo controllers C4. Servo controllers C5 that control the servomotors M5 to drive the front wheels (not shown) of the medical cart 3 are electrically connected to the positioner controller 31b. The encoders E5 that detect the rotation angles of the servomotors M5 are electrically connected to the servo controllers C5.

An operation command related to setting a preparation position, for example, is input from the input 33 to the positioner controller 31b. The positioner controller 31b generates position commands based on the operation command input from the input 33 and the rotation angles detected by the encoders E4, and outputs the position commands to the servo controllers C4. The servo controllers C4 generate torque commands based on the position commands input from the positioner controller 31b and the rotation angles detected by the encoders E4, and output the torque commands to the servomotors M4. Thus, the positioner 40 is moved according to the operation command input to the input 33. Similarly, the positioner controller 31b moves the medical cart 3 based on the operation command from the input 33.

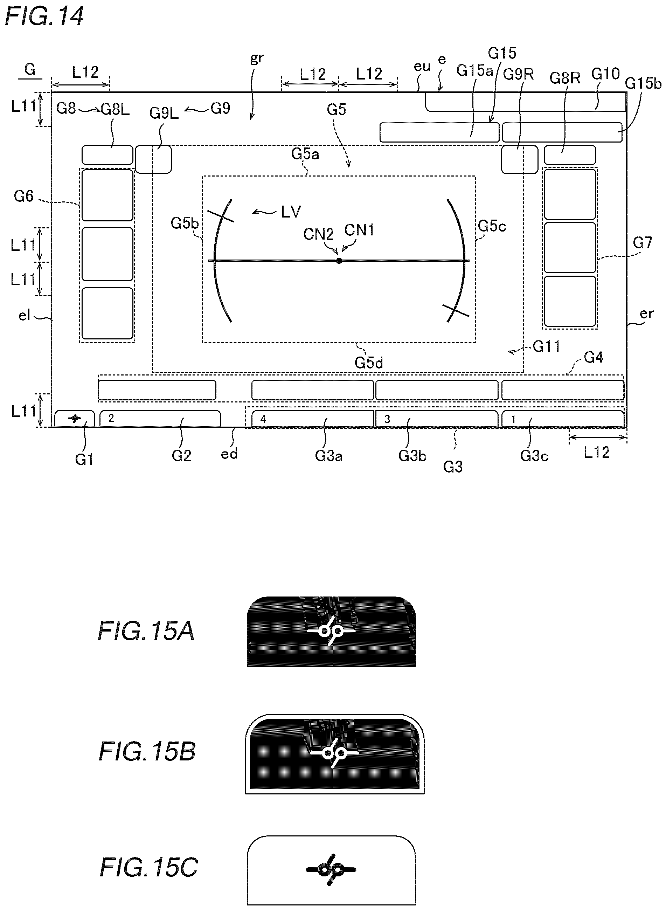

The surgical operation system 100 includes the image processor 8b. The image processor 8b executes processing based on a predetermined program. The image processor 8b includes a computer. The image processor 8b includes a processor such as a CPU that executes a program, and a storage such as a memory that stores the program. The image processor 8b generates a graphical user interface G (see FIG. 14) and displays, on the monitor 24 of the remote control apparatus 2, the graphical user interface G superimposed on the endoscopic image (see FIG. 13) captured by the endoscope 6. The image processor 8b also displays, on the display 8a, the graphical user interface G superimposed on the endoscopic image captured by the endoscope 6. The image processor 8b captures an image from the endoscope 6. The image processor 8b can communicate with the control unit 31.

As shown in FIG. 14, the graphical user interface G includes a clutch area G1. As shown in FIGS. 15A, 15B, and 15C, the state of the clutch pedal 22b is displayed in the clutch area G1. FIG. 15A shows a state (OFF state) in which the clutch pedal 22b is not pressed. FIG. 15B shows a state (hover state) in which the operator puts his/her foot on the clutch pedal 22b. FIG. 15C shows a state (ON state) in which the clutch pedal 22b is pressed.

As shown in FIG. 14, in this embodiment, the graphical user interface G includes a camera area G2 indicating information relating to the endoscope 6. The camera area G2 is displayed in an area in the vicinity of a lower end ed (see FIG. 13) of a screen gr of the monitor 24.

As shown in FIG. 14, the graphical user interface G includes hand areas G3. In the hand areas G3, information about the medical instruments 4, information about the arms 60, and information about the states of the coagulation pedals 22e and the incision pedals 22d are displayed. The hand areas G3 include a hand area G3a that indicates information about the medical instrument 4 and the arm 60a (number "4" of the hand areas G3) that are to be operated by the left-handed operation handle 21L, a hand area G3b that indicates information about the replacement medical instrument 4 and the arm 60b (number "3" of the hand areas G3), and a hand area G3c that indicates information about the medical instrument 4 and the arm 60d (number "1" of the hand areas G3) that are to be operated by the left-handed operation handle 21L. The hand areas G3 are displayed in an area in the vicinity of the lower end ed of the screen gr of the monitor 24. The clutch area G1 is also displayed in an area in the vicinity of the lower end ed of the screen gr. Specifically, as shown in FIG. 14, the clutch area G1, the camera area G2, and the hand areas G3 are displayed between the lower end ed of the screen gr and a position above the lower end ed of the screen gr by a length (L11) of one tenth of the vertical length of the screen gr.

The information about the arm 60 includes an arm number (arm number such as "1" or "2") of the arm 60 and an arrow icon that is displayed when the arm 60 is set as a replacement destination of an arm 60 to which the replacement medical instrument 4 has been attached. The information about the medical instrument 4 includes the name of the medical instrument 4. The information about the states of the coagulation pedals 22e and the incision pedals 22d includes the operation state of the clutch pedal 22b, the operation states of the incision pedals 22d, and the operation states of the coagulation pedals 22e.

As shown in FIG. 13, the hand areas (hand areas G3a and G3c) for the arms 60 that are targets to be operated are displayed in dark gray. In these dark gray hand areas G3a and G3c, the numbers "1" and "4" of the arms 60 that are the targets to be operated are displayed within white ovals. Furthermore, the hand area (hand area G3b) for the arm 60 that is not a target to be operated is displayed in light gray, and in this light gray hand area G3b, the number "3" of the arm 60 that is not a target to be operated is displayed in a lighter gray color than the light gray color of the hand area G3b.

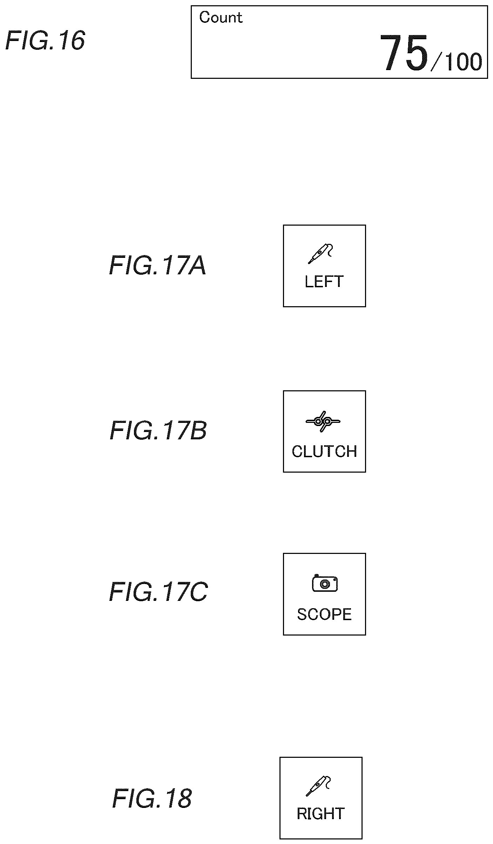

As shown in FIG. 14, the graphical user interface G includes a medical instrument usage information area G4, which is a pop-up area. In the medical instrument usage information area G4, the current number of uses/the maximum number of uses (see FIG. 16) of the medical instrument 4 attached to each arm 60 is displayed in a pop-up. When the current number of uses of the medical instrument becomes equal to the maximum number of uses of the medical instrument, the current number of uses is displayed in red. When an error occurs in the medical instrument 4 attached to any one of the arms 60, error information is displayed in a pop-up. When no medical instrument 4 is attached to any of the arms 60, nothing is displayed in the medical instrument usage information area G4. The medical instrument usage information area G4 is displayed in an area adjacent above the clutch area G1, the camera area G2, and the hand areas G3 on the monitor 24.

As shown in FIG. 14, the graphical user interface G includes a level indication area G5. In the level indication area G5, information about the angle of the endoscope 6 is displayed. The level indication area G5 is displayed only while the camera pedal 22c is being pressed. That is, when receiving a command that enables movement of the endoscope 6, the image processor 8b displays a level LV (level indication LV) of the endoscope 6 in the level indication area G5.

As shown in FIG. 14, the graphical user interface G includes a left pop-up area G6. In the left pop-up area G6, an icon shown in FIG. 17A, 17B, or 17C is displayed in a hover state in which the foot is placed on any of the foot pedals 22. FIG. 17A illustrates an icon displayed when the foot is placed on the coagulation pedal 22eL or the incision pedal 22dL. FIG. 17B illustrates an icon displayed when the foot is placed on the clutch pedal 22b. FIG. 17C illustrates an icon displayed when the foot is placed on the camera pedal 22c. The left pop-up area G6 is displayed in a left-side portion on the monitor 24.

As shown in FIG. 14, the graphical user interface G includes a right pop-up area G7. In the right pop-up area G7, an icon (FIG. 18) is displayed when the foot is placed on the coagulation pedal 22eR or the incision pedal 22dR. The right pop-up area G7 is displayed in a right-side portion on the monitor 24.

As shown in FIG. 14, the graphical user interface G includes first areas G8 to display the movable ranges of the arms 60, and the operable ranges of the arms 60 that can be operated by the operation handles 21 in the movable ranges of the arms 60. The graphical user interface G also includes second areas G9 to display the operation directions of the operation handles 21 required to return the operation handles 21 to within the operable ranges and/or ("and" in this embodiment) to return the arms 60 to within the movable ranges.

The number of arms 60 that can be operated by the operation handles 21 is two. For example, the operation handle 21L operates the left arm 60L (the arm 60a, for example; see FIG. 1) that supports the medical instrument 4. Furthermore, the operation handle 21R operates the right arm 60R (the arm 60d, for example; see FIG. 1) that supports the medical instrument 4. The first areas G8 (a first area G8L and a first area G8R) are provided separately for the left arm 60L and the right arm 60R, while the second areas G9 (a second area G9L and a second area G9R) are provided separately for the left arm 60L and the right arm 60R.

As shown in FIG. 14, the graphical user interface G includes error notification areas G15 (G15a, G15b). The error notification area G15a is displayed in a pop-up to indicate warning or error information when a warning or an error occurs. The error notification area G15b is displayed in a pop-up to indicate details of notes of the warning or the error displayed in the error notification area G15a.

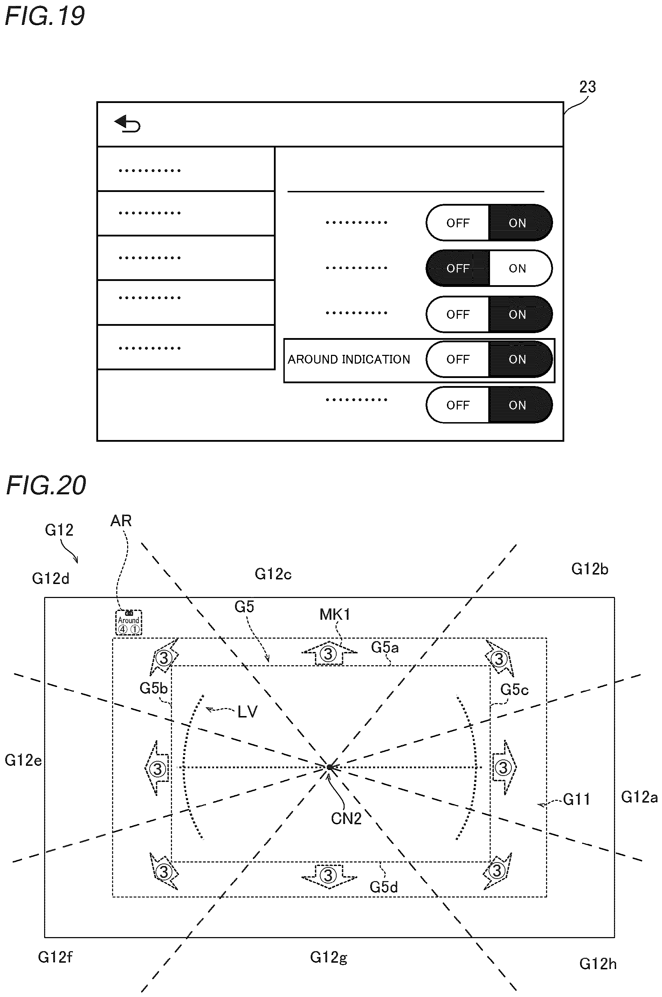

A around indication AR shown in FIG. 13, which indicates that the medical instrument 4 is located around an area within or outside the field of view of the endoscope 6, can be switched between a displayed state and a non-displayed state. Specifically, as shown in FIG. 19, the touch panel 23 of the remote control apparatus 2 is operated to display an "Around Indication" button. When "Around Indication" is selected as "ON", the around indication AR is displayed when the operator performs a predetermined input operation on the camera pedal 22c and the medical instrument 4 located around an area within or outside the field of view of the endoscope 6 is present. When "Around Indication" is selected as "OFF", the around indication AR is not displayed even when the operator performs the predetermined input operation on the camera pedal 22c. Thus, the display setting of the "Around Indication" can be easily switched depending on the operator's operating skill level and the operator's needs. A mark MK1 indicating that the medical instrument 4 is outside the field of view of the endoscope 6 may also be changed so as not to be displayed by the operator's settings.

As shown in FIG. 14, the graphical user interface G includes a status area G10. In the status area G10, information such as the remaining charge of a built-in battery of the medical manipulator 1, the brightness/contrast of the monitor 24, the lap time, and the elapsed time of the surgery is displayed. Specifically, as shown in FIG. 14, the status area G10 is displayed between an upper end eu of the screen gr and a position below the upper end eu of the screen gr by a length (L11) of one tenth of the vertical length of the screen gr.

The control unit 31 determines whether or not a plurality of medical instruments 4 are located outside the field of view of the endoscope 6 based on imaging range information of the endoscope 6 and position information of distal ends of the plurality of medical instruments 4 supported by the plurality of arms 60. That is, the control unit 31 acquires the positions of the medical instruments 4 based on the postures and positions of the arms 60. Furthermore, the control unit 31 acquires the imaging direction of the endoscope 6 based on the postures and positions of the arms 60. The control unit 31 also acquires the angle of view (field-of-view range) of the endoscope 6 based on the zooming state of the endoscope 6. The control unit 31 acquires the angle of view (field-of-view range) of the endoscope 6 using a value set as a mechanism (such as a lens) of the endoscope 6. Then, the control unit 31 acquires the coordinates of the distal ends of the medical instruments 4 with respect to the field of view of the endoscope 6 based on information about the field of view of the endoscope 6, the posture and position of the endoscope 6, and the positions of the arms 6. Thus, the control unit 31 determines whether or not the medical instruments 4 are located outside the field of view of the endoscope 6. The image processor 8b acquires information about the determination results as to whether or not the medical instruments 4 are located outside the field of view of the endoscope 6 from the control unit 31, and generates the graphical user interface G based on the acquired information.

As shown in FIGS. 13 and 14, when at least one of the plurality of medical instruments 4 is located outside the field of view of the endoscope 6, the image processor 8b displays, in an inner area of the graphical user interface G that does not include the vicinity of the edge of the screen of the display, the mark MK1 that indicates the medical instrument 4 that is located outside the field of view of the endoscope 6 in response to a predetermined input from the camera pedal 22c or the enable switch 81. That is, the image processor 8b acquires information that at least one of the plurality of medical instruments 4 is located outside the field of view of the endoscope 6 from the control unit 31, and generates and displays the graphical user interface G including the mark MK1 that indicates the medical instrument 4 located outside the field of view. The image processor 8b also displays the mark MK1 within an outer edge neighborhood area G11 of the level indication area G5 of the graphical user interface G. That is, the mark MK1 (the graphical user interface G) is displayed in the outer edge neighborhood area G11 (mark display area) of the level indication area G5 that includes a central portion CN1 of the screen gr of the monitor 24 but does not include the vicinity of an end e of the screen gr of the monitor 24. The outer edge neighborhood area G11 refers to an area located outside the level LV described below and adjacent to the level LV. The mark MK1 is an example of a "first indication" in the claims.

The image processor 8b displays the mark MK1 on the graphical user interface G in response to an operation on the enable switch 81 attached to the arm 60. In other words, when the operator such as an assistant or a nurse presses the enable switch 81 to move the arm 60, the mark MK1 is displayed on the graphical user interface G.

The mark MK1 includes an arrow that indicates a direction in which the medical instrument 4 located outside the field of view of the endoscope 6 is present. Specifically, a number ("3", for example) is displayed inside the arrow to identify the medical instrument 4 that is outside the field of view. The number may be located outside the arrow. The inside of the arrow is displayed transparently or semi-transparently such that an image can be seen through. For the medical instrument 4 to be operated, the number of the mark MK1 is indicated by the number of the arm 60 displayed in black color within a white circle. For the medical instrument 4 not to be operated, the number of the mark MK1 is indicated by the number of the arm 60 displayed in gray color within a black circle.

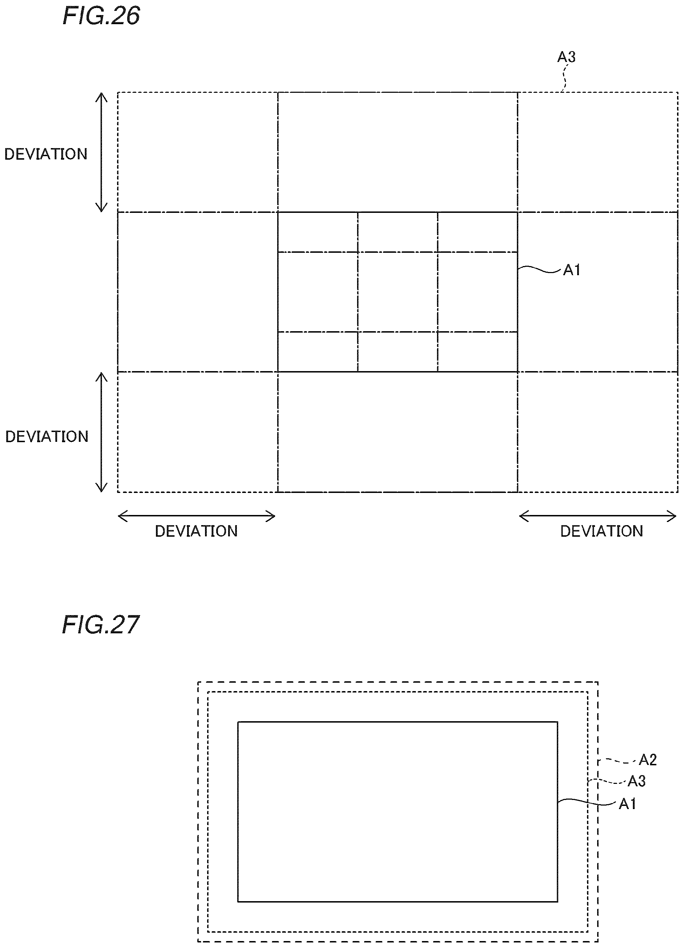

In this embodiment, as shown in FIG. 27, the control unit 31 acquires the distal end position of the medical instrument 4, and acquires a second imaging range A2 reflecting a positional deviation related to the distal end position of the medical instrument 4 and the diameter of the shaft of the medical instrument 4 with respect to a first imaging range A1 corresponding to the angle of view of the endoscope 6. Then, the control unit 31 determines whether or not the distal end position of the medical instrument 4 is within the second imaging range A2. In other words, the control unit 31 determines whether or not the distal end of the medical instrument 4 is within the second imaging range A2, which is wider than the first imaging range A1 corresponding to the angle of view of the endoscope. The distal end position of the medical instrument 4 includes a tool center point position (TCP) (see FIG. 6), which is a control center for moving the medical instrument 4.

As shown in FIG. 13, when the control unit 31 determines that the distal end position of the medical instrument 4 is outside the second imaging range A2, the image processor 8b displays the mark MK1 indicating that the medical instrument 4 is outside the field of view of the endoscope 6.

In this embodiment, when the control unit 31 determines that the distal end position of the medical instrument 4 is within the second imaging range A2, the image processor 8b displays the around indication AR indicating that the medical instrument 4 is located around an area within the field of view of the endoscope 6, or located around an area outside the field of view of the endoscope 6 in which the distal end position of the medical instrument 4 comes within the field of view when the endoscope 6 is pulled toward the proximal end side of the endoscope 6. The around indication AR is an example of a "second indication" in the claims.

When the control unit 31 determines that the distal end position of the medical instrument 4 is within the second imaging range A2, the image processor 8b displays an indication prompting the operator to pull the endoscope 6 toward the proximal end side of the endoscope 6. In other words, when the operator performs a predetermined input using the camera pedal 22c or the enable switch 81 and the around indication AR is displayed, the operator recognizes that even when the corresponding medical instrument 4 is not within the image of the endoscope 6, the corresponding medical instrument 4, which can be positioned within the image of the endoscope 6 by pulling the endoscope 6 toward the proximal end side of the endoscope 6, is present in any direction. Thus, the around indication AR prompts the operator to pull the endoscope 6 toward the proximal end side of the endoscope 6.

The around indication AR includes a number to identify the arm 60 supporting the medical instrument 4 determined to be within the second imaging range A2. In an example shown in FIG. 13, the around indication AR is displayed including a number "4" indicating that the medical instrument 4 supported by the arm 60a (a number "4" of the hand area G3) is located within the second imaging range A2, and a number "1" indicating that the medical instrument 4 supported by the arm 60d (a number "1" of the hand area G3) is located within the second imaging range A2.

As shown in FIG. 20, the image processor 8b displays the around indication AR in an area adjacent to the outer edge neighborhood area G11 of the level indication area G5 of the graphical user interface G. For example, the image processor 8b displays the around indication AR above the outer edge neighborhood area G11. The image processor 8b also displays the around indication AR in an area adjacent to the outside of the outer edge neighborhood area G11 in the right-left direction. For example, the image processor 8b displays the around indication AR on the left side of the outer edge neighborhood area G11 in the right-left direction.

When the control unit 31 determines that the distal end position of the medical instrument 4 is outside the second imaging range A2, the image processor 8b displays the mark MK1 indicating that the medical instrument 4 is outside the field of view of the endoscope 6 in response to the predetermined input to the camera pedal 22c or the enable switch 81. When the control unit 31 determines that the distal end position of the medical instrument 4 is within the second imaging range A2, the image processor 8b displays the around indication AR indicating that the medical instrument 4 is located around the area within the field of view of the endoscope 6, or located around the area outside the field of view of the endoscope 6 in which the medical instrument 4 comes within the field of view when the endoscope 6 is pulled toward the proximal end side of the endoscope 6, in response to the predetermined input to the camera pedal 22c or the enable switch 81.

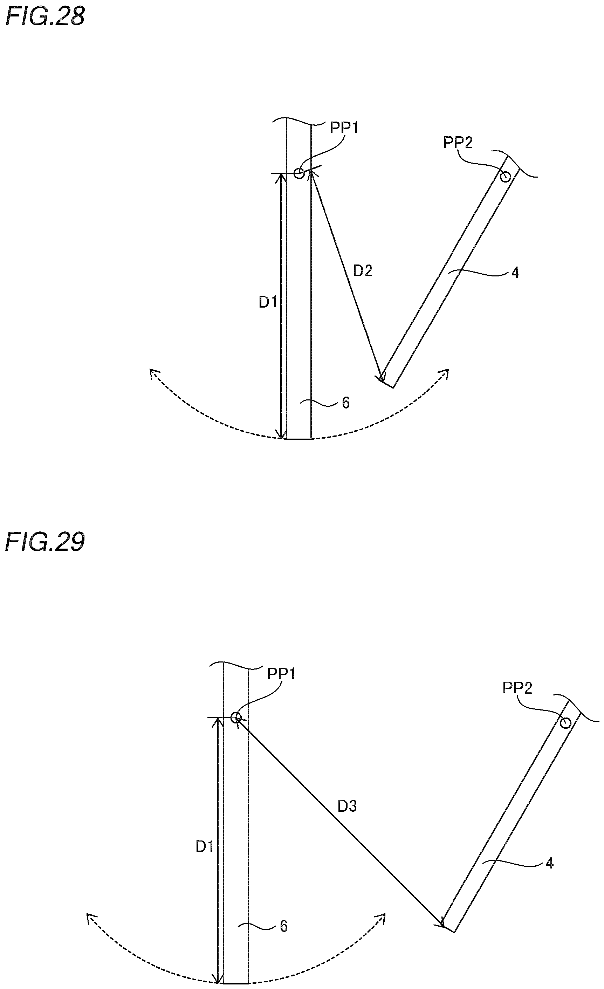

As shown in FIG. 28, the control unit 31 acquires the distal end positions of the endoscope 6 and the medical instrument 4. The control unit 31 also determines whether or not a distance D2 between the distal end position of the medical instrument 4 and a pivot position PP1 is greater than a distance D1 between the distal end position of the endoscope 6 and the pivot position PP1. When the control unit 31 determines that the distance D2 between the distal end position of the medical instrument 4 and the pivot position PP1 is less than the distance D1 between the distal end position of the endoscope 6 and the pivot position PP1, the image processor 8b displays an indication prompting the operator to pull the endoscope 6 toward the proximal end side of the endoscope 6.

The image processor 8b displays the around indication AR indicating that the medical instrument 4 is located around an area outside the field of view of the endoscope 6 in which the distal end position of the medical instrument 4 comes within the field of view when the endoscope 6 is pulled toward the proximal end side of the endoscope 6, as the indication prompting the operator to pull the endoscope 6 toward the proximal end side of the endoscope 6.

As shown in FIG. 29, when the distal end position of the medical instrument 4 is on the proximal end side of the endoscope 6 with respect to the distal end position of the endoscope 6, the image processor 8b displays an indication of the direction of the distal end position of the medical instrument 4 with respect to the imaging range of the endoscope 6 (displays the mark MK1) when the control unit 31 determines that the distance between the distal end position of the medical instrument 4 and the pivot position PP1 is greater than the distance between the distal end position of the endoscope 6 and the pivot position PP1.

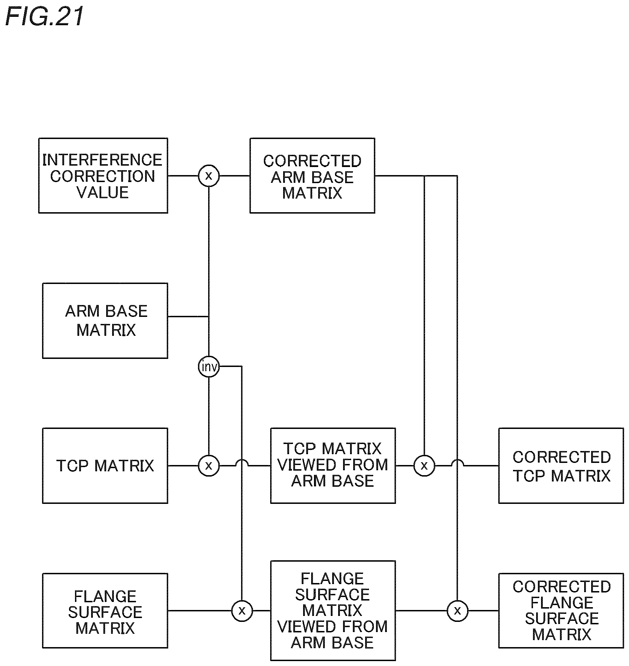

The control unit 31 corrects a positional deviation caused by an error in a distance between the arm 60c and the arm 60a, 60b, or 60d, and acquires the distal end positions of the endoscope 6 and the medical instrument 4. For example, as shown in FIG. 21, the control unit 31 acquires a corrected arm base matrix based on an interference correction value and an arm base matrix. Furthermore, the control unit 31 acquires a TCP matrix viewed from the arm base based on the arm base matrix and a TCP matrix. Moreover, the control unit 31 acquires a flange surface matrix viewed from the arm base based on the arm base matrix and a flange surface matrix. Then, the control unit 31 acquires a corrected TCP matrix based on the corrected arm base matrix and the TCP matrix viewed from the arm base. Furthermore, the control unit 31 acquires a corrected flange surface matrix based on the corrected arm base matrix and the flange surface matrix viewed from the arm base.

The positional deviation related to the distal end position of the medical instrument 4 includes a first positional deviation caused by a positional deviation of the field-of-view range of the endoscope 6, and a second positional deviation that is a positional deviation of the distal end of the medical instrument 4. Then, the control unit 31 calculates the first positional deviation based on a positional deviation of the endoscope 6 at the pivot position PP1 and the distance of the distal end of the endoscope 6 from the pivot position PP1. Furthermore, the control unit 31 calculates the second positional deviation based on a positional deviation of the medical instrument 4 at a pivot position PP2 and the distance of the distal end of the medical instrument 4 from the pivot position PP2.

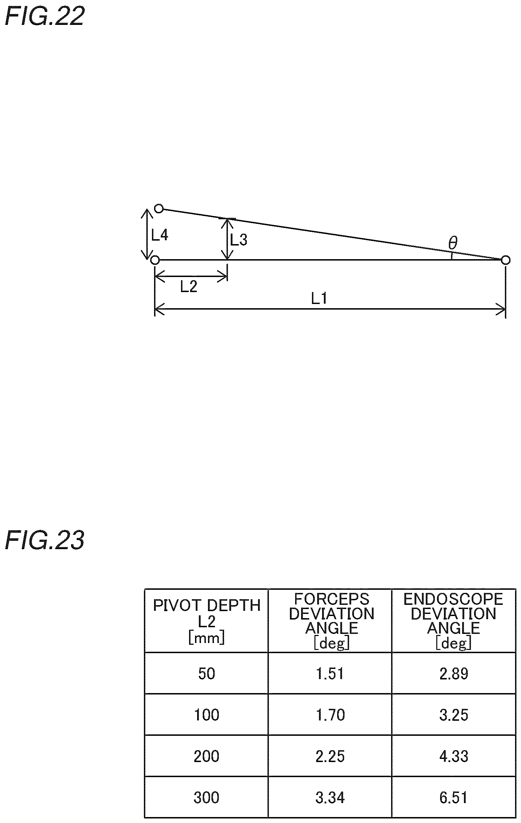

Specifically, as shown in FIG. 22, the control unit 31 calculates a deviation amount based on the length L1 of the shaft of the medical instrument 4 (endoscope 6), the distance (pivot depth) L2 of the distal end of the medical instrument 4 (endoscope 6) from the pivot position PP, and a deviation radius L3. The taught pivot position PP is the inner circumferential surface of the trocar T, and thus a deviation occurs from the actual pivot position PP by the radius of the medical instrument 4 (endoscope 6). Therefore, the deviation radius L3 is a length corresponding to the radius of the medical instrument 4 (endoscope 6). Then, a deviation angle θ is calculated based on L1, L2, and L3. The deviation angle θ is calculated by θ = atan(L3/(L1 - L2)). L1 and L3 are constant values for each medical instrument 4 (endoscope 6), and thus the deviation angle θ changes when the distance (pivot depth) L2 of the distal end of the medical instrument 4 (endoscope 6) from the pivot position PP changes.

For example, the deviation angle θ varies with the change in the pivot depth L2, as shown in FIG. 23. In an example shown in FIG. 23, the length L1 of the shaft of the medical instrument 4 (pair of forceps) is 507 mm, and the deviation radius L3 is 4 mm. Furthermore, the length L1 of the shaft of the endoscope 6 is 498 mm, and the deviation radius L3 is 6 mm.

A deviation amount L4 at the field-of-view position of the endoscope 6 is calculated by L4 = (L1 + depth from the endoscope 6 to the medical instrument 4) × sin θ. As shown in FIG. 22, the deviation amount L4 at the distal end position (TCP position) of the medical instrument 4 can be calculated by L4 = L1 × sin θ.

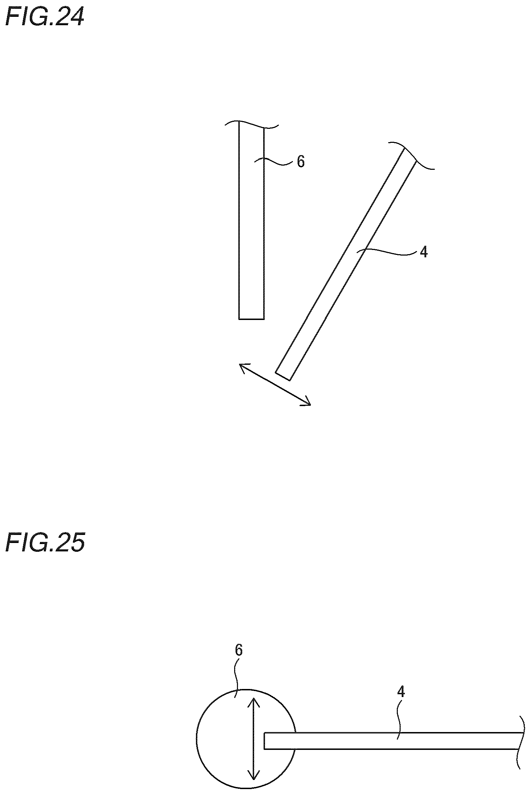

The influence of the deviation of the distal end of the medical instrument 4 on the field of view of the endoscope 6 varies depending on the insertion direction of the medical instrument 4 with respect to a field-of-view direction, and thus correction is performed. A direction of deviation is different when the medical instrument 4 is inserted obliquely with respect to the field of view of the endoscope 6 as shown in FIG. 24 and when the medical instrument 4 is inserted laterally with respect to the field of view of the endoscope 6 as shown in FIG. 25. For example, when the medical instrument 4 is inserted at a right angle from the side with respect to the field of view of the endoscope 6, the position of the medical instrument 4 does not deviate in the lateral direction of the field of view (the insertion direction of the medical instrument 4). The insertion direction of the medical instrument 4 is calculated based on two points: the TCP position of the medical instrument 4 and a position shifted by a shaft offset amount from the flange surface.

Based on the deviation amount of the medical instrument 4 in the insertion direction, the deviation amount of the medical instrument 4 in a 90-degree direction with respect to the insertion direction, the deviation amount of the medical instrument 4 in a 45-degree direction with respect to the insertion direction, and the deviation amount of the medical instrument 4 at -45 degrees with respect to the insertion direction, the deviation amount with respect to the field of view is obtained, and the maximum values of the vertical and horizontal fields of view are used as the vertical and horizontal deviation amounts for correction.

As shown in FIG 26, the positional deviation related to the distal end position of the medical instrument 4 with respect to the first imaging range A1 corresponding to the angle of view of the endoscope 6 includes the first positional deviation caused by the positional deviation of the field-of-view range of the endoscope 6, and the second positional deviation that is the positional deviation of the distal end of the medical instrument 4. The control unit 31 calculates a third imaging range A3 taking into consideration the positional deviation related to the distal end position of the medical instrument 4. When the deviation amount is large, a direction outside the field of view may not be able to be determined (the direction outside the field of view may change depending on the direction of deviation).

The calculation of the third imaging range A3 with respect to the first imaging range A1 is performed separately for each of the plurality of medical instruments 4. That is, the deviation amount of each of the plurality of medical instruments 4 is calculated from the insertion distances and insertion angles of the plurality of medical instruments 4.

As shown in FIG. 27, the second imaging range A2 reflecting the diameter of the shaft of the medical instrument 4 with respect to the third imaging range A3 taking into consideration the positional deviation of the field-of-view range of the endoscope 6 and the positional deviation of the distal end position of the medical instrument 4 with respect to the first imaging range A1 is acquired. The second imaging range A2 is a range that is larger vertically and horizontally than the third imaging range A3 by the diameter of the shaft of the medical instrument 4. When the point of the distal end position (TCP position) of the medical instrument 4 falls within the second imaging range A2, the distal end of the medical instrument 4 is considered to be within the field-of-view range of the endoscope 6.

In the example shown in FIGS. 13 and 14, the medical instruments 4 (the medical instruments 4 such as the pairs of forceps 4b other than the endoscope 6) are respectively attached to the arm 60a (corresponding to the hand area G3a having the number "4"), the arm 60b (corresponding to the hand area G3b having the number "3"), the arm 60d (corresponding to the hand area G3c having the number "1") among the arms 60a, 60b, 60c, and 60d. The endoscope 6 is attached to the arm 60c (corresponding to the camera area G2 having the number "2"). The arm 60a (corresponding to the hand area G3a having the number "4") and the arm 60d (corresponding to the hand area G3c having the number "1") are in active states in which the arm 60a and 60d are allowed to be operated by the operation handles 21, and the hand areas G3a and G3c are displayed in dark gray. To the contrary, the arm 60b is in an inactive state in which the arm 60b is not allowed to be operated by the operation handles 21, and the hand area G3b is displayed in light gray.

In the example shown in FIG. 13, the medical instruments 4 supported by the arm 60a (corresponding to the hand area G3a having the number "4") and the medical instrument 4 supported by the arm 60d (corresponding to the hand area G3c having the number "1") are located in the field of view of the endoscope 6. To the contrary, the medical instrument 4 supported by the arm 60b (corresponding to the hand area G3b having the number "3") is located outside the field of view of the endoscope 6.

As shown in FIG. 13, the image processor 8b displays the mark MK1 in the direction in which the medical instrument 4 outside the field of view is located in the outer edge neighborhood area G11 adjacent to the level indication area G5. That is, the graphical user interface G is displayed by arranging the mark MK1 indicating the medical instrument 4 outside the field of view in the outer edge neighborhood area G11 and in an area G12 (see FIG. 20) corresponding to the direction in which the medical instrument 4 outside the field of view is located with respect to a center CN2 of the level indication area G5. In FIG. 13, the mark MK1 (an arrow and a character "3") is displayed.

As shown in FIG. 20, the area G12 in which the mark MK1 indicating the medical instrument 4 outside the field of view is displayed is one of areas G12 obtained by dividing the outer edge neighborhood area G11 into a plurality of areas radially from the center CN2 of the level indication area G5. The outer edge neighborhood area G11 is divided into eight areas radially from the center CN2. In FIG. 13, the mark MK1 (an arrow and a character "3") is displayed in an upper area G12c (see FIG. 20) as an example. In FIG. 20, all patterns are displayed in which the medical instrument 4 is located outside the field of view.

As shown in FIG. 20, the image processor 8b displays the mark MK1 indicating the direction corresponding to the area in which the medical instrument 4 outside the field of view is located, among the eight areas outside the field of view that are divided according to the shape of the rectangular display, the vertical and horizontal lengths of which are not equal to each other.

As shown in FIG. 20, the display has a horizontal length greater than its vertical length. The area outside the field of view is divided into eight areas according to the shape of the display such that the angles of the upper area G12c and a lower area G12g are greater than the angles of a right area G12a, a left area G12e, an upper right area G12b, an upper left area G12d, a lower right area G12h, and a lower left area G12f.

The image processor 8b displays the mark MK1 at a predetermined position within each of the eight divided areas of the outer edge vicinity area G11.

As shown in FIG. 14, the image processor 8b displays the level LV of the endoscope 6 in the level indication area G5 when the image processor 8b receives a command to enable movement of the endoscope 6 via the control unit 31. That is, while a camera movement operation is being performed on the camera pedal 22c, the image processor 8b displays the level of the endoscope 6 in the level indication area G5 of the graphical user interface G. Then, the image processor 8b displays, on the monitor 24, the graphical user interface G for displaying the mark MK1 in the outer edge neighborhood area G11 outside the level indication area G5. The level LV represents the inclination of the field of view of the endoscope 6 with respect to the patient P.

As shown in FIG. 14, the level indication area G5 is a rectangular area (horizontally long rectangle) including an upper side G5a, a left side G5b, a right side G5c, and a lower side G5d. Thus, the mark MK1 is displayed in the outer edge neighborhood area G11 of the level indication area G5 of a predetermined size including the central portion of the screen gr such that the visibility of the operator can be improved. The upper side G5a is located between a position above the central portion CN1 of the screen gr of the monitor 24 by a length (L11) of one tenth of the vertical length of the screen gr and a position below the upper end eu of the screen gr by a length (L11) of one tenth of the vertical length of the screen gr. Preferably, the upper side G5a is located between a position above the central portion CN1 of the screen gr of the monitor 24 by a length of one eighth of the vertical length of the screen gr and a position below the upper end eu of the screen gr by a length of one eighth of the vertical length of the screen gr. More preferably, the upper side G5a is located between a position above the central portion CN1 of the screen gr of the monitor 24 by a length of one sixth of the vertical length of the screen gr and a position below the upper end eu of the screen gr by a length of one sixth of the vertical length of the screen gr.

The upper side G5a is located below the status area G10 in which the remaining charge of the built-in battery of the medical manipulator 1 or the like is displayed.

The left side G5b is located between a position on the left side of the central portion CN1 of the screen gr of the monitor 24 by a length (L12) of one tenth of the horizontal length of the screen gr and a position on the right side of a left end el of the screen gr by a length (L12) of one tenth of the horizontal length of the screen gr. Preferably, the left side G5b is located between a position on the left side of the central portion CN1 of the screen gr of the monitor 24 by a length of one eighth of the horizontal length of the screen gr and a position on the right side of the left end el of the screen gr by a length of one eighth of the horizontal length of the screen gr. More preferably, the left side G5b is located between a position on the left side of the central portion CN1 of the screen gr of the monitor 24 by a length of one sixth of the horizontal length of the screen gr and a position on the right side of the left end el of the screen gr by a length of one sixth of the horizontal length of the screen gr.

The right side G5c is located between a position on the right side of the central portion CN1 of the screen gr of the monitor 24 by a length (L12) of one tenth of the horizontal length of the screen gr and a position on the left side of a right end er of the screen gr by a length (L12) of one tenth of the horizontal length of the screen gr. Preferably, the right side G5c is located between a position on the right side of the central portion CN1 of the screen gr of the monitor 24 by a length of one eighth of the horizontal length of the screen gr and a position on the left side of the right end er of the screen gr by a length of one eighth of the horizontal length of the screen gr. More preferably, the right side G5c is located between a position on the right side of the central portion CN1 of the screen gr of the monitor 24 by a length of one sixth of the horizontal length of the screen gr and a position on the left side of the right end er of the screen gr by a length of one sixth of the horizontal length of the screen gr.

The lower side G5d is located between a position below the central portion CN1 of the screen gr of the monitor 24 by a length (L11) of one tenth of the vertical length of the screen gr and a position above the lower end ed of the screen gr by a length (L11) of one tenth of the vertical length of the screen gr. Preferably, the lower side G5d is located between a position below the central portion CN1 of the screen gr of the monitor 24 by a length of one eighth of the vertical length of the screen gr and a position above the lower end ed of the screen gr by a length of one eighth of the vertical length of the screen gr. More preferably, the lower side G5d is located between a position below the central portion CN1 of the screen gr of the monitor 24 by a length of one sixth of the vertical length of the screen gr and a position above the lower end ed of the screen gr by a length of one sixth of the vertical length of the screen gr.

The lower side G5d of the level indication area G5 is located above the hand area G3a, the hand area G3b, the hand area G3c, and the camera area G2. Furthermore, the lower side G5d of the level indication area G5 is located above the medical instrument usage information area G4.

The central portion CN1 of the screen gr of the monitor 24 and the center CN2 of the level indication area G5 are at substantially the same position.

The mark MK1 indicating the medical instrument 4 located outside the field of view is located between the level indication area G5 and at least one (all in this embodiment) of the medical instrument usage information area G4, the left pop-up area G6, the right pop-up area G7, and the status area G10. The medical instrument usage information area G4 indicates the state of the medical instrument 4. The left pop-up area G6 is displayed when any of the foot pedals 22 is operated (in a hover state in which the foot is placed on any of the foot pedals 22). The right pop-up area G7 is displayed when the coagulation pedal 22eR or the incision pedal 22dR is operated (when the foot of the operator is placed on either pedal). The status area G10 indicates the state of the surgical operation system 100.

As shown in FIG. 13, the mark MK1 includes a number to identify the arm 60 that supports the medical instrument 4 that is located outside the field of view. In the example shown in FIG. 13, the mark MK1 is displayed including the number "3" that indicates that the medical instrument 4 supported by the arm 60b (number "3" of the hand area G3) is located outside the field of view of the endoscope 6.

### Out-Of-Field-Of-View Determination Process

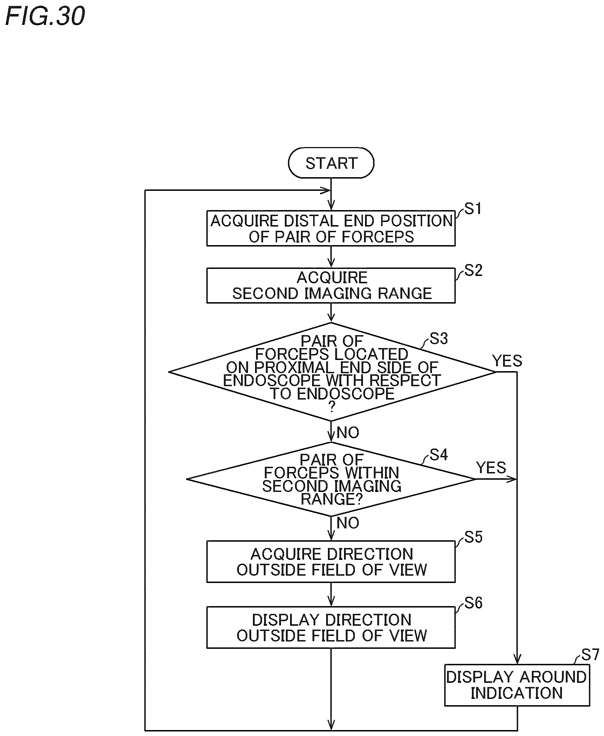

An out-of-field-of-view determination process in the surgical operation system 100 is now described with reference to FIG. 30. The out-of-field-of-view determination process is performed by the control unit 31.

When a predetermined input is performed through the camera pedal 22c or the enable switch 81, in step S1, the control unit 31 acquires the distal end position of the medical instrument 4 (pair of forceps). In step S2, the control unit 31 acquires the second imaging range A2.

In step S3, the control unit 31 determines whether or not the medical instrument 4 (pair of forceps) is located on the proximal end side of the endoscope 6 with respect to the endoscope 6. When the medical instrument 4 is located on the proximal end side, the process advances to step S7, and when the endoscope 6 is located on the proximal end side of the endoscope 6 with respect to the medical instrument 4, the process advances to step S4. In step S4, the control unit 31 determines whether or not the medical instrument 4 (pair of forceps) is within the second imaging range A2. When the medical instrument 4 (pair of forceps) is within the second imaging range A2, the process advances to step S7, and when the medical instrument 4 (pair of forceps) is outside the second imaging range A2, the process advances to step S5.

In step S5, the control unit 31 acquires the direction outside the field of view. In step S6, the image processor 8b displays the direction outside the field of view using the mark MK1. Then, the process returns to step S1. In step S7, the image processor 8b displays the around indication AR. Then, the process returns to step S1.

### Advantages of This Embodiment

According to this embodiment, the following advantages are achieved.

According to this embodiment, as described above, the control unit 31 is configured or programmed to acquire the distal end position of the medical instrument 4, acquire the second imaging range A2 reflecting the positional deviation related to the distal end position of the medical instrument 4 and the diameter of the shaft of the medical instrument 4 with respect to the first imaging range A1 corresponding to the angle of view of the endoscope 6, and determine whether or not the distal end position of the medical instrument 4 is within the second imaging range A2. Accordingly, whether or not the distal end position of the medical instrument 4 is outside the field of view of the endoscope 6 can be determined based on the second imaging range A2 reflecting the positional deviation related to the distal end position of the medical instrument 4. Consequently, when the distal end position of the medical instrument 4 with respect to the field of view of the endoscope 6 is uncertain due to the distal end position of the medical instrument 4 being deviated with respect to the field of view of the endoscope 6, the possibility is reduced or prevented that the direction of the uncertain distal end position of the medical instrument 4 is indicated. Furthermore, whether or not the distal end position of the medical instrument 4 is outside the field of view of the endoscope 6 can be determined based on the second imaging range A2 reflecting the diameter of the shaft of the medical instrument 4, and thus it is possible to determine whether or not the distal end position of the medical instrument 4 having some size, not just a specific point at the distal end of the medical instrument 4, is within the field of view of the endoscope 6. Consequently, even when the medical instrument 4 is positionally deviated, the operator can easily confirm the position of the medical instrument 4.

According to this embodiment, as described above, the image processor 8b is configured or programmed to display the mark MK1 indicating that the medical instrument 4 is outside the field of view of the endoscope 6 when the control unit 31 determines that the distal end position of the medical instrument 4 is outside the second imaging range A2. Accordingly, when the distal end position of the medical instrument 4 is definitely outside the field of view of the endoscope 6, the mark MK1 is displayed, and thus the operator can easily find the distal end position of the medical instrument 4 by pointing the endoscope 6 in a direction indicated by the display of the mark MK1.

According to this embodiment, as described above, the image processor 8b is configured or programmed to display the around indication AR indicating that the medical instrument 4 is located around the area within the field of view of the endoscope 6, or located around the area outside the field of view of the endoscope 6 in which the distal end position of the medical instrument 4 comes within the field of view when the endoscope 6 is pulled toward the proximal end side of the endoscope 6 when the control unit 31 determines that the distal end position of the medical instrument 4 is within the second imaging range A2. Accordingly, when the distal end of the medical instrument 4 is located around the field of view of the endoscope 6, the around indication AR is displayed, and thus the around indication AR enables the operator to easily recognize that the distal end of the medical instrument 4 is located around the field of view. Furthermore, when the distal end of the medical instrument 4 is not reflected in the field of view of the endoscope 6, the operator can easily find the distal end position of the medical instrument 4 by pulling the endoscope 6 toward the proximal end side of the endoscope 6.

According to this embodiment, as described above, the image processor 8b is configured or programmed to display an indication prompting the operator to pull the endoscope 6 toward the proximal end side of the endoscope 6 when the control unit 31 determines that the distal end position of the medical instrument 4 is within the second imaging range A2. Accordingly, when the distal end of the medical instrument 4 is not reflected in the field of view of the endoscope 6, the operator can be prompted to pull the endoscope 6 toward the proximal end side of the endoscope 6.

According to this embodiment, as described above, the around indication AR includes a number to identify the arm 60 supporting the medical instrument 4 determined to be within the second imaging range A2. Accordingly, the operator can easily identify and recognize the medical instrument 4 that is around the field of view of the endoscope 6.

According to this embodiment, as described above, the image processor 8b is configured or programmed to, in response to the predetermined input to the camera pedal 22c or the enable switch 81, display the mark MK1 indicating that the medical instrument 4 is outside the field of view of the endoscope 6 when the control unit 31 determines that the distal end position of the medical instrument 4 is outside the second imaging range A2. Furthermore, the image processor 8b is configured or programmed to, in response to the predetermined input to the camera pedal 22c or the enable switch 81, display the around indication AR indicating that the medical instrument 4 is located around the area within the field of view of the endoscope 6, or located around the area outside the field of view of the endoscope 6 in which the medical instrument 4 comes within the field of view when the endoscope 6 is pulled toward the proximal end side of the endoscope 6 when the control unit 31 determines that the distal end position of the medical instrument 4 is within the second imaging range A2. Accordingly, when the operator performs the predetermined input to the camera pedal 22c or the enable switch 81, the mark MK1 or the around indication AR is displayed, and thus the distal end position of the medical instrument 4 can be easily confirmed by the operation of the operator.

According to this embodiment, as described above, the positional deviation related to the distal end position of the medical instrument 4 includes the first positional deviation caused by the positional deviation range of the field of view of the endoscope 6 and the second positional deviation that is the positional deviation of the distal end of the medical instrument 4. Accordingly, both the positional deviation of the field-of-view range of the endoscope 6 and the positional deviation of the distal end of the medical instrument 4 can be treated together as the positional deviation related to the distal end position of the medical instrument 4.

According to this embodiment, as described above, the control unit 31 is configured or programed to calculate the first positional deviation based on the positional deviation of the endoscope 6 at the pivot position PP1 and the distance of the distal end of the endoscope 6 from the pivot position PP1. Accordingly, the first positional deviation can be easily calculated based on the insertion distance of the endoscope 6. Furthermore, the control unit 31 is configured or programed to calculate the second positional deviation based on the positional deviation of the medical instrument 4 at the pivot position PP2 and the distance of the distal end of the medical instrument 4 from the pivot position PP2. Accordingly, the second positional deviation can be easily calculated based on the insertion distance of the medical instrument 4.

According to this embodiment, as described above, the distal end position of the medical instrument 4 includes a tool center point position (TCP), which is a control center for operating the medical instrument 4. Accordingly, the operator can easily know the tool center point position, which is a control center when the medical instrument 4 is operated by the operator.

According to this embodiment, as described above, the image processor 8b is configured or programmed to display an indication prompting the operator to pull the endoscope 6 toward the proximal end side of the endoscope 6 when the control unit 31 determines that the distance between the distal end position of the medical instrument 4 and the pivot position PP1 is smaller than the distance between the distal end position of the endoscope 6 and the pivot position PP1. Accordingly, when the distance between the distal end position of the medical instrument 4 and the pivot position PP1 is smaller than the distance between the distal end position of the endoscope 6 and the pivot position PP1, and the distal end position of the medical instrument 4 does not enter the field of view of the endoscope 6 even when the orientation of the endoscope 6 is changed, an indication is displayed prompting the operator to pull the endoscope 6 toward the proximal end side of the endoscope 6. Thus, the operator can easily find the distal end position of the medical instrument 4 outside the field of view by pulling the endoscope 6 toward the proximal end side of the endoscope 6. Consequently, even when the medical instrument 4 is positionally deviated, the operator can easily confirm the position of the medical instrument 4.

According to this embodiment, as described above, the image processor 8b is configured or programmed to display the around indication AR indicating that the medical instrument 4 is located around the area outside the field of view of the endoscope 6 in which the distal end position of the medical instrument 4 comes within the field of view when the endoscope 6 is pulled toward the proximal end side of the endoscope 6, as the indication prompting the operator to pull the endoscope 6 toward the proximal end side of the endoscope 6. Accordingly, when the distal end of the medical instrument 4 is not reflected in the field of view of the endoscope 6, the operator can easily recognize that the medical instrument 4 is located around the area outside the field of view in which the distal end position of the medical instrument 4 comes within the field of view when the endoscope 6 is pulled toward the proximal end side of the endoscope 6.

According to this embodiment, as described above, the image processor 8b is configured or programmed to, when the distal end position of the medical instrument 4 is on the proximal end side of the endoscope 6 with respect to the distal end position of the endoscope 6, display an indication of the direction of the distal end position of the medical instrument 4 with respect to the imaging range of the endoscope 6 when the control unit 31 determines that the distance between the distal end position of the medical instrument 4 and the pivot position PP1 is greater than the distance between the distal end position of the endoscope 6 and the pivot position PP1. Accordingly, when the orientation of the endoscope 6 is changed such that the distal end position of the medical instrument 4 enters the imaging range of the endoscope 6, the operator can easily recognize the distal end position of the medical instrument 4.

According to this embodiment, as described above, the control unit 31 is configured or programmed to correct the positional deviation caused by the error in the distance between the arm 60c and the arm 60a, 60b or 60d, and acquire the distal end positions of the endoscope 6 and the medical instrument 4. Accordingly, the distal end positions of the endoscope 6 and the medical instrument 4 can be accurately acquired by correcting positional deviations caused by assembly errors when the arms 60a, 60b, 60c, and 60d are assembled.

### Modified Examples

The embodiment disclosed this time must be considered as illustrative in all points and not restrictive. The scope of the present invention is not shown by the above description of the embodiment but by the scope of claims for patent, and all modifications (modified examples) within the meaning and scope equivalent to the scope of claims for patent are further included.

For example, while the example in which the image processor 8b captures an image from the endoscope 6 and generates the graphical user interface G has been shown in the aforementioned embodiment, the present invention is not limited to this. For example, the control unit 31 of the medical manipulator 1 may generate the graphical user interface G, or a control unit (not shown) of the remote control apparatus 2 may generate the graphical user interface G. Alternatively, an image processor that captures an image from the endoscope 6 and performs image processing, and an image processor that generates the graphical user interface G and superimposes the graphical user interface G on the image from the endoscope 6 may be provided separately.

While the example in which the control unit 31 of the medical manipulator 1 acquires the distal end position of the medical instrument 4, acquires the second imaging range A2 reflecting the positional deviation related to the distal end position of the medical instrument 4 and the diameter of the shaft of the medical instrument 4 with respect to the first imaging range A1 corresponding to the angle of view of the endoscope 6, and determines whether or not the distal end position of the medical instrument 4 is within the second imaging range A2 has been shown in the aforementioned embodiment, the present invention is not limited to this. In the present invention, for example, the image processor 8b or a control unit (not shown) of the remote control apparatus 2 may acquire the distal end position of the medical instrument 4, acquire the second imaging range A2 reflecting the positional deviation related to the distal end position of the medical instrument 4 and the diameter of the shaft of the medical instrument 4 with respect to the first imaging range A1, and determine whether or not the distal end position of the medical instrument 4 is within the second imaging range A2. Alternatively, a plurality of control units (such as the control unit 31, the image processor 8b, and the remote control apparatus 2) may perform the above processing.

While the example in which the image processor 8b determines whether or not an input has been performed through the camera pedal 22c or the enable switch 81 has been shown in the aforementioned embodiment, the present invention is not limited to this. For example, the control unit 31 may determine whether or not an input has been performed through the camera pedal 22c or the enable switch 81, and transmit the determination result to the image processor 8b.

While the example in which when it is determined that the distal end position of the medical instrument 4 is within the second imaging range A2, the around indication AR is displayed to indicate that the medical instrument 4 is located around the area within the field of view of the endoscope 6, or located around the area outside the field of view of the endoscope 6 in which the distal end position of the medical instrument 4 comes within the field of view when the endoscope 6 is pulled toward the proximal end side of the endoscope 6 has been shown in the aforementioned embodiment, the present invention is not limited to this. In the present invention, an indication indicating that the medical instrument 4 is located around the area within the field of view of the endoscope 6, or located around the area outside the field of view of the endoscope 6 in which the distal end position of the medical instrument 4 comes within the field of view when the endoscope 6 is pulled toward the proximal end side of the endoscope 6 may be displayed as "PULL" or "ZOOM OUT," which directly instructs the operator to pull the endoscope 6. Alternatively, a graphic indication may be displayed to prompt the operator to pull the endoscope 6.

While the example in which when it is determined that the distal end position of the medical instrument 4 is outside the second imaging range A2, the mark MK1 including an arrow indicating that the medical instrument 4 is outside the field of view of the endoscope 6 is displayed has been shown in the aforementioned embodiment, the present invention is not limited to this. In the present invention, the mark indicating that the medical instrument 4 is outside the field of view of the endoscope 6 may not include an arrow.

While the example in which the plurality of arms 60 include the enable switches 81, the joysticks 82, and the switch units 83 has been shown in the aforementioned embodiment, the present invention is not limited to this. For example, at least one of the plurality of arms 60 may include the enable switch 81, the joystick 82, and the switch unit 83.

While the example in which in response to the enable switch 81 of the arm 60 being operated, the around indication AR and the mark MK1 are displayed on the graphical user interface G has been shown in the aforementioned embodiment, the present invention is not limited to this. For example, the around indication AR and the mark MK1 may be displayed on the graphical user interface G in response to another switch (the joystick 82 or the switch unit 83) of the arm 60 being operated.

While the example in which when the predetermined input is performed through the camera pedal 22c or the enable switch 81, the around indication AR and the mark MK1 are displayed on the graphical user interface G has been shown in the aforementioned embodiment, the present invention is not limited to this. For example, even when the predetermined input is performed through another input, the around indication AR and the mark MK1 may be displayed on the graphical user interface G. For example, when the predetermined input is performed through a switch (first switch) provided on the operation handle 21, the around indication AR and the mark MK1 may be displayed on the graphical user interface G.

While the example in which when one or two medical instruments 4 are located outside the field of view of the endoscope 6, the mark MK1 is arranged closer to the center of the screen gr has been shown in the aforementioned embodiment, the present invention is not limited to this. For example, a position at which the mark MK1 corresponding to each of the plurality of medical instruments 4 is arranged may be fixed.

While the example in which the distal end position of the medical instrument 4, which is to be determined whether to be within the second imaging range A2, is the tool center point has been shown in the aforementioned embodiment, the present invention is not limited to this. In the present invention, the distal end of the medical instrument 4 may be the distal ends of the end effector members 104a and 104b.

While the example in which each of the arm portion 61 and the positioner 40 includes a 7-axis articulated robot has been shown in the aforementioned embodiment, the present invention is not limited to this. For example, each of the arm portion 61 and the positioner 40 may include an articulated robot having an axis configuration (six axes or eight axes, for example) other than the 7-axis articulated robot.

While the example in which the medical manipulator 1 includes the medical cart 3, the positioner 40, and the arm base 50 has been shown in the aforementioned embodiment, the present invention is not limited to this. For example, the medical manipulator 1 may not include the medical cart 3, the positioner 40, or the arm base 50, but may include only the arms 60.

The functionality of the elements disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out the recited functionality or hardware that is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor.

### Aspects

It will be appreciated by those skilled in the art that the exemplary embodiments described above are specific examples of the following aspects.

### (Item 1)

A surgical system comprising:
a first manipulator to support an endoscope;
a second manipulator to support a surgical instrument; and
a controller including one or more processors; wherein
the controller is configured or programmed to acquire a distal end position of the surgical instrument, acquire a second imaging range reflecting a positional deviation related to the distal end position of the surgical instrument and a diameter of a shaft of the surgical instrument with respect to a first imaging range corresponding to an angle of view of the endoscope, and determine whether or not the distal end position of the surgical instrument is within the second imaging range.

### (Item 2)

The surgical system according to item 1, wherein the controller is configured or programmed to display a first indication indicating that the surgical instrument is outside a field of view of the endoscope when it is determined that the distal end position of the surgical instrument is outside the second imaging range.

### (Item 3)

The surgical system according to item 1 or 2, wherein the controller is configured or programmed to display a second indication indicating that the surgical instrument is located around an area within a field of view of the endoscope, or located around an area outside the field of view of the endoscope in which the distal end position of the surgical instrument comes within the field of view when the endoscope is pulled toward a proximal end side of the endoscope 6 when it is determined that the distal end position of the surgical instrument is within the second imaging range.

### (Item 4)

The surgical system according to item 3, wherein the controller is configured or programmed to display, as the second indication, an indication prompting an operator to pull the endoscope toward the proximal end side of the endoscope when it is determined that the distal end position of the surgical instrument is within the second imaging range.

### (Item 5)

The surgical system according to item 4, wherein the second indication includes a number to identify the second manipulator supporting the surgical instrument determined to be within the second imaging range.

### (Item 6)

The surgical system according to any one of items 1 to 5, wherein the controller is configured or programmed to, in response to a predetermined input to an input, display a first indication indicating that the surgical instrument is outside a field of view of the endoscope when it is determined that the distal end position of the surgical instrument is outside the second imaging range, and display a second indication indicating that the surgical instrument is located around an area within the field of view of the endoscope, or located around an area outside the field of view of the endoscope in which the surgical instrument comes within the field of view when the endoscope is pulled toward a proximal end side of the endoscope when it is determined that the distal end position of the surgical instrument is within the second imaging range.

### (Item 7)

The surgical system according to item 6, wherein the input includes at least one of a first switch on an operation handle to operate the surgical instrument, a second switch on the second manipulator, or a third switch to move the endoscope using the operation handle.

### (Item 8)

The surgical system according to any one of items 1 to 7, wherein the positional deviation related to the distal end position of the surgical instrument includes a first positional deviation caused by a positional deviation of a field-of-view range of the endoscope and a second positional deviation that is a positional deviation of a distal end of the surgical instrument.

### (Item 9)

The surgical system according to item 8, wherein the controller is configured or programed to calculate the first positional deviation based on a positional deviation of the endoscope at a pivot position of the endoscope and a distance of a distal end of the endoscope from the pivot position of the endoscope, and calculate the second positional deviation based on a positional deviation of the surgical instrument at a pivot position of the surgical instrument and a distance of the distal end of the surgical instrument from the pivot position of the surgical instrument.

### (Item 10)

The surgical system according to any one of items 1 to 9, wherein the distal end position of the surgical instrument includes a tool center point position.

### (Item 11)

A surgical system comprising:
a first manipulator to support an endoscope;
a second manipulator to support a surgical instrument; and
a controller including one or more processors; wherein
the controller is configured or programmed to control the first manipulator to move the endoscope about a first pivot position as a fulcrum, and control the second manipulator to move the surgical instrument about a second pivot position as a fulcrum; and
the controller is configured or programmed to acquire a distal end position of each of the endoscope and the surgical instrument, and display an indication prompting an operator to pull the endoscope toward a proximal end side of the endoscope when a distance between the distal end position of the surgical instrument and the first pivot position is smaller than a distance between the distal end position of the endoscope and the first pivot position.

### (Item 12)

The surgical system according to item 11, wherein the controller is configured or programmed to display an indication indicating that the surgical instrument is located around an area outside a field of view of the endoscope in which the distal end position of the surgical instrument comes within the field of view when the endoscope is pulled toward the proximal end side of the endoscope, as the indication prompting the operator to pull the endoscope toward the proximal end side of the endoscope.

### (Item 13)

The surgical system according to item 11 or 12, wherein the controller is configured or programmed to, when the distal end position of the surgical instrument is on the proximal end side of the endoscope with respect to the distal end position of the endoscope, display an indication of a direction of the distal end position of the surgical instrument with respect to an imaging range of the endoscope when the distance between the distal end position of the surgical instrument and the first pivot position is greater than the distance between the distal end position of the endoscope and the first pivot position.

### (Item 14)

The surgical system according to any one of items 11 to 13, wherein the controller is configured or programmed to correct a positional deviation caused by an error in a distance between the first manipulator and the second manipulator, and acquire the distal end position of each of the endoscope and the surgical instrument.

### Description of Reference Numerals

4: medical instrument (surgical instrument)
6: endoscope
8b: image processor (controller)
21, 21L, 21R: operation handle
22c: camera pedal (input, third switch)
31: control unit (controller)
60c: arm (first manipulator)
60a, 60b, 60d: arm (second manipulator)
81: enable switch (input, second switch)
100: surgical operation system (surgical system)
A1: first imaging range
A2: second imaging range
AR: around indication (second indication)
MK1: mark (first indication)
PP1: pivot position (first pivot position)
PP2: pivot position (second pivot position)

## Claims

1. A surgical system comprising:
a first manipulator to support an endoscope;
a second manipulator to support a surgical instrument; and
a controller including one or more processors;
wherein
the controller is configured or programmed to acquire a distal end position of the surgical instrument, acquire a second imaging range reflecting a positional deviation related to the distal end position of the surgical instrument and a diameter of a shaft of the surgical instrument with respect to a first imaging range corresponding to an angle of view of the endoscope, and determine whether or not the distal end position of the surgical instrument is within the second imaging range.

2. The surgical system according to claim 1, wherein the controller is configured or programmed to display a first indication indicating that the surgical instrument is outside a field of view of the endoscope when it is determined that the distal end position of the surgical instrument is outside the second imaging range.

3. The surgical system according to claim 1, wherein the controller is configured or programmed to display a second indication indicating that the surgical instrument is located around an area within a field of view of the endoscope, or located around an area outside the field of view of the endoscope in which the distal end position of the surgical instrument comes within the field of view when the endoscope is pulled toward a proximal end side of the endoscope when it is determined that the distal end position of the surgical instrument is within the second imaging range.

4. The surgical system according to claim 3, wherein the controller is configured or programmed to display, as the second indication, an indication prompting an operator to pull the endoscope toward the proximal end side of the endoscope when it is determined that the distal end position of the surgical instrument is within the second imaging range.

5. The surgical system according to claim 4, wherein the second indication includes a number to identify the second manipulator supporting the surgical instrument determined to be within the second imaging range.

6. The surgical system according to claim 1, wherein the controller is configured or programmed to, in response to a predetermined input to an input, display a first indication indicating that the surgical instrument is outside a field of view of the endoscope when it is determined that the distal end position of the surgical instrument is outside the second imaging range, and display a second indication indicating that the surgical instrument is located around an area within the field of view of the endoscope, or located around an area outside the field of view of the endoscope in which the surgical instrument comes within the field of view when the endoscope is pulled toward a proximal end side of the endoscope when it is determined that the distal end position of the surgical instrument is within the second imaging range.

7. The surgical system according to claim 6, wherein the input includes at least one of a first switch on an operation handle to operate the surgical instrument, a second switch on the second manipulator, or a third switch to move the endoscope using the operation handle.

8. The surgical system according to claim 1, wherein the positional deviation related to the distal end position of the surgical instrument includes a first positional deviation caused by a positional deviation of a field-of-view range of the endoscope and a second positional deviation that is a positional deviation of a distal end of the surgical instrument.

9. The surgical system according to claim 8, wherein the controller is configured or programed to calculate the first positional deviation based on a positional deviation of the endoscope at a pivot position of the endoscope and a distance of a distal end of the endoscope from the pivot position of the endoscope, and calculate the second positional deviation based on a positional deviation of the surgical instrument at a pivot position of the surgical instrument and a distance of the distal end of the surgical instrument from the pivot position of the surgical instrument.

10. The surgical system according to claim 1, wherein the distal end position of the surgical instrument includes a tool center point position.

11. A surgical system comprising:
a first manipulator to support an endoscope;
a second manipulator to support a surgical instrument; and
a controller including one or more processors;
wherein
the controller is configured or programmed to control the first manipulator to move the endoscope about a first pivot position as a fulcrum, and control the second manipulator to move the surgical instrument about a second pivot position as a fulcrum; and
the controller is configured or programmed to acquire a distal end position of each of the endoscope and the surgical instrument, and display an indication prompting an operator to pull the endoscope toward a proximal end side of the endoscope when a distance between the distal end position of the surgical instrument and the first pivot position is smaller than a distance between the distal end position of the endoscope and the first pivot position.

12. The surgical system according to claim 11, wherein the controller is configured or programmed to display an indication indicating that the surgical instrument is located around an area outside a field of view of the endoscope in which the distal end position of the surgical instrument comes within the field of view when the endoscope is pulled toward the proximal end side of the endoscope, as the indication prompting the operator to pull the endoscope toward the proximal end side of the endoscope.

13. The surgical system according to claim 11, wherein the controller is configured or programmed to, when the distal end position of the surgical instrument is on the proximal end side of the endoscope with respect to the distal end position of the endoscope, display an indication of a direction of the distal end position of the surgical instrument with respect to an imaging range of the endoscope when the distance between the distal end position of the surgical instrument and the first pivot position is greater than the distance between the distal end position of the endoscope and the first pivot position.

14. The surgical system according to claim 11, wherein the controller is configured or programmed to correct a positional deviation caused by an error in a distance between the first manipulator and the second manipulator, and acquire the distal end position of each of the endoscope and the surgical instrument.
